# EUROPEAN PATENT APPLICATION

(11) **EP 4 393 515 A1**
(43) Date of publication of application: **03.07.2024**
(21) Application number: 22860649.7
(22) Date of filing: 26.08.2022
(51) Int. Cl.: A61K 47/68, C07K 16/28, C07K 16/30, A61K 39/395, A61P 35/00

(54) **ANTI-CLDN-18.2 ANTIBODY-DRUG CONJUGATE AND USE THEREOF**

(30) Priority: 26.08.2021 CN 202110989043
(71) Applicant: Shanghai Junshi Biosciences Co., Ltd., Pilot Free Trade Zone Shanghai 201210 (CN)
(72) Inventor: HUANG, Guofeng, Suzhou, Jiangsu 215003 (CN); CHEN, Si, Suzhou, Jiangsu 215003 (CN); GENG, Jingbo, Suzhou, Jiangsu 215003 (CN); ZHANG, Jing, Suzhou, Jiangsu 215003 (CN); FU, Cexiong, Suzhou, Jiangsu 215003 (CN); LIU, Hongchuan, Suzhou, Jiangsu 215003 (CN); ZHOU, Yuehua, Suzhou, Jiangsu 215003 (CN); YAO, Sheng, Shanghai 201210 (CN); FENG, Hui, Suzhou, Jiangsu 215003 (CN)
(74) Representative: Cabinet Becker et Associés
(86) International application number: PCT/CN2022/115237
(87) International publication number: WO 2023/025303

(57) **Abstract**

Provided are an anti-CLDN-18.2 antibody-drug conjugate that specifically binds to CLDN-18.2 and a composition comprising same. Also provided are a method for using the antibody-drug conjugate of the present invention and a use thereof.

## Description

### Technical Field

The present invention provides an anti-CLDN-18.2 antibody-drug conjugate that specifically binds to CLDN-18.2 and a composition comprising same. Also provided are a method for using the antibody-drug conjugate of the present invention and a use thereof.

### Background Art

Gastric cancer is one of the most prevalent cancers worldwide. According to statistics from the World Health Organization's cancer control project, up to 7 million patients die of cancer worldwide each year, of which 700,000 patients die from gastric cancer. Compared with conventional gastric cancer treatment regimens, antibody-based treatment regimens have far-reaching application prospects due to their high specificity and low side effects.

Claudin, also known as CLDN, is a family of cell surface proteins that establish paracellular barriers and control the flow of molecules between cells. At least 26 Claudin species have been discovered so far. Members of the Claudin protein family are important structural components of tight junctions, which play an important role in maintaining epithelial cell polarity, controlling paracellular diffusion, and regulating cell growth and differentiation. Claudin molecules cross the cell membrane four times, with both the N-terminus and the C-terminus in the cytoplasm. Different Claudin members are expressed in different tissues, and alterations in their function are associated with cancer formation. Changes in the expression levels of Claudin 1, Claudin 18, and Claudin 10 are associated with intestinal cancer, gastric cancer, and hepatocellular carcinoma, respectively.

Claudin 18 (CLDN18) has two alternative splicing variants, CLDN-18.1 and CLDN-18.2. Claudin 18.1 (CLDN-18.1) is selectively expressed in normal lung and gastric epithelium. Claudin 18.2 (CLDN-18.2) shows trace expression in normal gastric epithelial short-lived cells, but in tumor cells, Claudin 18.2 shows strong expression in various cancer types, for example, Claudin 18.2 is highly expressed in 75% of patients with gastric cancer, 50% of patients with pancreatic cancer and 30% of patients with esophageal cancer, and also in lung cancer and so on.

Antibody-drug conjugate (ADC) consists of three parts: an antibody or an antigen-binding fragment thereof (targeting), a linker and a small molecule drug. Antibodies or antigen-binding fragments thereof are conjugated to small molecule drugs with biological activity such as cytotoxicity, such as cytotoxins, via cleavable or non-cleavable linkers, which makes full use of the specificity of antibodies or antigen-binding fragments thereof when targeting cells of interest (targeted cells) or binding to high-expression antigens and the high efficiency of small molecule drugs, and reduces or avoids the toxic and side effects on non-targeted cells. This means that compared with a traditional tumor chemotherapy drug, the antibody-drug conjugate for tumors can accurately target tumor cells and reduce the impact on non-tumor cells.

The anti-CLDN-18.2 antibody-drug conjugate IMAB-362-vcMMAE developed by Ganymed is currently in the preclinical stage.

There is still a need in the art for an anti-CLDN-18.2 antibody-drug conjugate with excellent affinity, specificity, *etc.*

### Summary of the Invention

The present invention provides a novel anti-CLDN-18.2 antibody-drug conjugate, which has the advantages of high affinity and high specificity for human CLDN-18.2. The anti-CLDN-18.2 antibody-drug conjugate provided by the present invention can be used as an independent therapy or in combination with other therapies or other anti-cancer agents for the treatment of, for example, a cancer.

The present invention provides an anti-CLDN-18.2 antibody-drug conjugate (ADC) with a structure shown in formula (I-1), or a pharmaceutically acceptable salt thereof:

Ab-(L-D)m formula (I-1)

wherein,
L is a linker unit;
D is a small molecule drug with cytotoxicity;
m represents the average number of L-D units conjugated to Ab, and m is selected from 1-8, preferably 2-7, more preferably 2-5;
Ab is an anti-CLDN-18.2 antibody or an antigen-binding fragment thereof, which comprises
   (I) a heavy chain variable region comprising HCDR1 and HCDR2 with amino acid sequences as shown in SEQ ID NO: 1 and SEQ ID NO: 72, respectively, and HCDR3 as shown in SEQ ID NO: 73 or 3; and a light chain variable region comprising LCDR1, LCDR2 and LCDR3 with amino acid sequences as shown in SEQ ID NO: 7, SEQ ID NO: 8 and SEQ ID NO: 9, respectively; or
   (II) a heavy chain variable region comprising HCDR1, HCDR2 and HCDR3 with amino acid sequences as shown in SEQ ID NO: 1, SEQ ID NO: 41 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
   (III) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
   (IV) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 19, SEQ ID NO: 20 and SEQ ID NO: 21, respectively; or
   (V) a heavy chain variable region comprising HCDR1 and HCDR2 with amino acid sequences as shown in SEQ ID NO: 28 and SEQ ID NO: 74, respectively, and HCDR3 as shown in SEQ ID NO: 30 or 75; and a light chain variable region comprising LCDR1, LCDR2 and LCDR3 with amino acid sequences as shown in SEQ ID NO: 13, SEQ ID NO: 14 and SEQ ID NO: 15, respectively.

In some embodiments, the small molecule drug D of the present invention is monomethyl auristatin or maytansine alkaloids; preferably, the monomethyl auristatin is monomethyl auristatin E (MMAE) or monomethyl auristatin F (MMAF), and the maytansine is N2'-deacetyl-N2'-(3-mercapto-1-oxopropyl)-maytansine (DM1), N2'-deacetyl-N2'-(4-mercapto-1-oxopentyl)-maytansine (DM3) or N2'-deacetyl-N2'-(4-mercapto-4-methyl-1-oxopentyl)-maytansine (DM4).

In some embodiments, L of the present invention is selected from N-succinimidyl 4-(2-pyridyldithio)pentanoate (SPP), N-succinimidyl (4-iodoacetyl)aminobenzoate (SIAB), N-succinimidyl 4-(maleimidomethyl)cyclohexanecarboxylate (SMCC), 6-maleimidocaproyl (MC), maleimidopropionyl (MP), valine-citrulline (VC), alanine-phenylalanine (ala-phe), p-aminobenzyloxycarbonyl (PAB) and MC-VC-PAB (maleimidocaproyl-valine-citrulline-p-aminobenzyloxycarbonyl); preferably, the L is 6-maleimidocaproyl (MC) or MC-VC-PAB.

In some embodiments, the anti-CLDN-18.2 antibody-drug conjugate of the present invention is represented by the following formula: 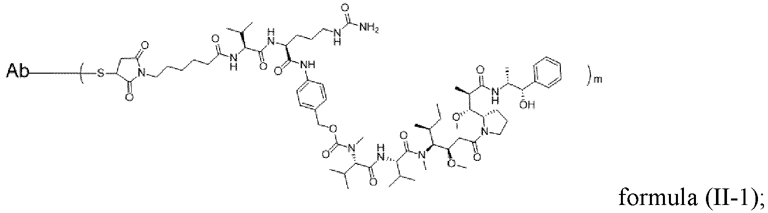 or 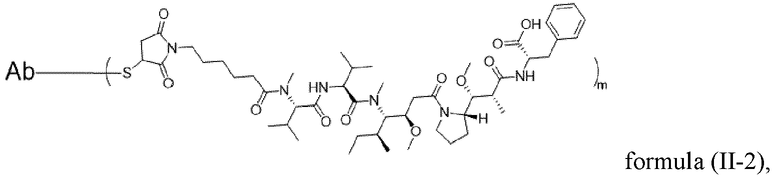 wherein m and Ab are as defined in claim 1.

In some embodiments, the anti-CLDN-18.2 antibody or antigen-binding fragment thereof of the present invention comprises:
(I) a heavy chain variable region with an amino acid sequence as shown in or having at least 95%, 96%, 97%, 98% or 99% identity to any one of SEQ ID NO: 55, 57, 82 or 83, and a light chain variable region with an amino acid sequence as shown in or having at least 95%, 96%, 97%, 98% or 99% identity to SEQ ID NO: 56; or
(II) a heavy chain variable region with an amino acid sequence as shown in or having at least 95%, 96%, 97%, 98% or 99% identity to any one of SEQ ID NO: 60, 76 or 78, and a light chain variable region with an amino acid sequence as shown in or having at least 95%, 96%, 97%, 98% or 99% identity to SEQ ID NO: 61; or
(III) a heavy chain variable region with an amino acid sequence as shown in or having at least 95%, 96%, 97%, 98% or 99% identity to SEQ ID NO: 76, and a light chain variable region with an amino acid sequence as shown in or having at least 95%, 96%, 97%, 98% or 99% identity to SEQ ID NO: 77; or
(IV) a heavy chain variable region with an amino acid sequence as shown in or having at least 95%, 96%, 97%, 98% or 99% identity to SEQ ID NO: 79; and a light chain variable region with an amino acid sequence as shown in or having at least 95%, 96%, 97%, 98% or 99% identity to SEQ ID NO: 80; or
(V) a heavy chain variable region with an amino acid sequence as shown in or having at least 95%, 96%, 97%, 98% or 99% identity to SEQ ID NO: 55, and a light chain variable region with an amino acid sequence as shown in or having at least 95%, 96%, 97%, 98% or 99% identity to SEQ ID NO: 81.

In some embodiments, the anti-CLDN-18.2 antibody or antigen-binding fragment thereof of the present invention comprises:
(1) a heavy chain with an amino acid sequence as shown in or having at least 95%, 96%, 97%, 98% or 99% identity to SEQ ID NO: 64; and a light chain with an amino acid sequence as shown in or having at least 95%, 96%, 97%, 98% or 99% identity to SEQ ID NO: 65; or
(2) a heavy chain with an amino acid sequence as shown in or having at least 95%, 96%, 97%, 98% or 99% identity to SEQ ID NO: 68; and a light chain with an amino acid sequence as shown in or having at least 95%, 96%, 97%, 98% or 99% identity to SEQ ID NO: 69; or
(3) a heavy chain with an amino acid sequence as shown in or having at least 95%, 96%, 97%, 98% or 99% identity to SEQ ID NO: 84; and a light chain with an amino acid sequence as shown in or having at least 95%, 96%, 97%, 98% or 99% identity to SEQ ID NO: 85; or
(4) a heavy chain with an amino acid sequence as shown in or having at least 95%, 96%, 97%, 98% or 99% identity to SEQ ID NO: 86; and a light chain with an amino acid sequence as shown in or having at least 95%, 96%, 97%, 98% or 99% identity to SEQ ID NO: 87; or
(5) a heavy chain with an amino acid sequence as shown in or having at least 95%, 96%, 97%, 98% or 99% identity to SEQ ID NO: 88; and a light chain with an amino acid sequence as shown in or having at least 95%, 96%, 97%, 98% or 99% identity to SEQ ID NO: 89; or
(6) a heavy chain with an amino acid sequence as shown in or having at least 95%, 96%, 97%, 98% or 99% identity to SEQ ID NO: 90; and a light chain with an amino acid sequence as shown in or having at least 95%, 96%, 97%, 98% or 99% identity to SEQ ID NO: 91; or
(7) a heavy chain with an amino acid sequence as shown in or having at least 95%, 96%, 97%, 98% or 99% identity to SEQ ID NO: 92; and a light chain with an amino acid sequence as shown in or having at least 95%, 96%, 97%, 98% or 99% identity to SEQ ID NO: 93; or
(8) a heavy chain with an amino acid sequence as shown in or having at least 95%, 96%, 97%, 98% or 99% identity to SEQ ID NO: 94; and a light chain with an amino acid sequence as shown in or having at least 95%, 96%, 97%, 98% or 99% identity to SEQ ID NO: 95; or
(9) a heavy chain with an amino acid sequence as shown in or having at least 95%, 96%, 97%, 98% or 99% identity to SEQ ID NO: 96; and a light chain with an amino acid sequence as shown in or having at least 95%, 96%, 97%, 98% or 99% identity to SEQ ID NO: 97; or
(10) a heavy chain with an amino acid sequence as shown in or having at least 95%, 96%, 97%, 98% or 99% identity to SEQ ID NO: 98; and a light chain with an amino acid sequence as shown in or having at least 95%, 96%, 97%, 98% or 99% identity to SEQ ID NO: 99; or
(11) a heavy chain with an amino acid sequence as shown in or having at least 95%, 96%, 97%, 98% or 99% identity to SEQ ID NO: 100; and a light chain with an amino acid sequence as shown in or having at least 95%, 96%, 97%, 98% or 99% identity to SEQ ID NO: 101.

In some embodiments, the anti-CLDN-18.2 antibody or antigen-binding fragment thereof of the present invention comprises:
(I) a heavy chain with an amino acid sequence as shown in SEQ ID NO: 64 and a light chain with an amino acid sequence as shown in SEQ ID NO: 65; or
(II) a heavy chain with an amino acid sequence as shown in SEQ ID NO: 68 and a light chain with an amino acid sequence as shown in SEQ ID NO: 69.

In some embodiments, the antibody or antigen-binding fragment of the present invention is a murine antibody, a chimeric antibody, a humanized antibody, or a fully human antibody.

In some embodiments, the anti-CLDN-18.2 antibody or antigen-binding fragment thereof of the present invention is Fab, Fab', Fab'-SH, Fv, scFv, F(ab')2 or sdAb.

In some embodiments, the anti-CLDN-18.2 antibody or antigen-binding fragment thereof of the present invention is any IgG subtype, such as IgG1, IgG2, IgG3 or IgG4; preferably, the antibody is hypofucosylated or afucosylated.

In some embodiments, the anti-CLDN-18.2 antibody or antigen-binding fragment thereof of the present invention is hypofucosylated.

In some embodiments, an anti-CLDN-18.2 antibody or antigen-binding fragment thereof of the present invention is afucosylated.

In yet another aspect, the present invention provides a pharmaceutical composition comprising the anti-CLDN-18.2 antibody-drug conjugate or the pharmaceutically acceptable salt thereof as described herein, and a pharmaceutically acceptable carrier or excipient.

In yet another aspect, the present invention provides use of the antibody-drug conjugate or pharmaceutically acceptable salt thereof as described herein, or the pharmaceutical composition as described herein in the preparation of a medicament for the treatment and/or prevention of a disease or condition mediated by CLDN-18.2; preferably, the disease or condition is a cancer.

In yet another aspect, the present invention provides a method of treating and/or preventing a disease or condition mediated by CLDN-18.2, comprising administering the antibody-drug conjugate as described herein, or the pharmaceutical composition as described herein to a subject in need thereof; preferably, the disease or condition is a cancer.

In yet another aspect, the present invention provides an antibody-drug conjugate as described herein or a pharmaceutical composition as described herein, for use in the treatment and/or prevention of a disease or condition mediated by CLDN-18.2; preferably, the disease or condition is a cancer.

In some embodiments, the cancer of the present invention is selected from gastric cancer, esophageal cancer, gastroesophageal cancer, pancreatic cancer, cholangiocarcinoma, lung cancer, ovarian cancer, colon cancer, liver cancer, head and neck cancer, gallbladder cancer, bowel cancer, and bladder cancer.

In yet another aspect, the present invention provides a pharmaceutical combination comprising the antibody-drug conjugate or pharmaceutically acceptable salt thereof as described herein or the pharmaceutical composition as described herein, and one or more additional therapeutic agents.

In yet another aspect, the present invention provides a kit comprising the antibody-drug conjugate as described herein, or the pharmaceutical composition as described herein.

### Brief Description of the Drawings

Figs. 1a, 1b, 1c and 1d: affinity of anti-CLDN-18.2 antibody-drug conjugate to human CLDN-18.2 overexpressed on the surface of gastric cancer cell line NUGC4-CLDN-18.2 in a cell experiment.
Figs. 2a and 2b: endocytic activity of anti-CLDN-18.2 antibody-drug conjugate into gastric cancer cell line NUGC4-CLDN-18.2 overexpressing human CLDN-18.2 in a cell experiment.
Figs. 3a and 3c: anti-CLDN-18.2 antibody-drug conjugate inhibits the proliferation of gastric cancer cell line NUGC4-CLDN-18.2 cells overexpressing human CLDN-18.2; Fig. 3b: humanized anti-CLDN-18.2 antibody-drug conjugate inhibits the proliferation of wild-type gastric cancer cell line NUGC4 cells.
Fig. 4: anti-tumor effect of anti-CLDN-18.2 antibody-drug conjugate in the NUGC4 CLDN-18.2 subcutaneous transplanted tumor model.
Fig. 5: anti-tumor effect of anti-CLDN-18.2 antibody-drug conjugate in the NUGC4 CLDN-18.2 subcutaneous transplanted tumor model.
Fig. 6: anti-tumor effect of anti-CLDN-18.2 antibody-drug conjugate in the GA0006 subcutaneous transplanted tumor model.

### Detailed Description of Embodiments

### Definitions

The practice of the present invention may employ, unless otherwise indicated, conventional techniques of molecular biology (including recombinant techniques), microbiology, cell biology, biochemistry, and immunology, which are within the skill of the art.

In order to understand the present invention more easily, certain technical terms are specifically defined as follows. Unless otherwise explicitly defined elsewhere herein, all technical and scientific terms used herein have the meanings commonly understood by those of ordinary skill in the art to which the present invention belongs. For definitions and terms in this field, professionals can refer to Current Protocols in Molecular Biology (Ausubel). Abbreviations for amino acid residues are the standard 3-letter and/or 1-letter codes used in the art to refer to one of the 20 commonly used L-amino acids. As used herein (including the claims), singular forms include their corresponding plural forms unless the context explicitly dictates otherwise.

The term "about" when used in connection with a numerical value is meant to encompass numerical values within a range having a lower limit that is 5% smaller than the specified numerical value and an upper limit that is 5% larger than the specified numerical value.

The term "and/or" should be understood as meaning any one of the optional items or a combination of any two or more of the optional items.

The term "CLDN-18.2" or "Claudin18.2", is one of the two splicing variants of Claudin 18. The term refers to any native CLDN-18.2 from any vertebrate, including mammals such as primates (for example, human), and rodents (for example, mice and rats), unless otherwise stated. The term encompasses "full-length" unprocessed CLDN-18.2 as well as any form of CLDN-18.2 or any fragment thereof produced by intracellular processing. The term also includes naturally occurring variants of CLDN-18.2, for example, splicing variants or allelic variants. In a preferred embodiment, CLDN-18.2 refers to full-length CLDN-18.2 or a fragment thereof (such as a mature fragment thereof lacking a signal peptide) from human and cynomolgus monkey.

The term "percent (%) amino acid sequence identity" or simply "identity" is defined as the percentage of amino acid residues in a candidate amino acid sequence that are identical to the amino acid residues in the reference amino acid sequence, after aligning the amino acid sequences and introducing gaps, if necessary, to achieve the maximum percent sequence identity, and not considering any conservative substitutions as part of the sequence identity. Alignment of sequences for the purpose of determining percent amino acid sequence identity can be achieved by using a variety of methods in the art, for instance, using publicly available computer software, such as BLAST, BLAST-2, ALIGN or MEGALIGN (DNASTAR) software. A person skilled in the art can determine appropriate parameters for measuring alignment, including any algorithms needed to achieve maximal alignment over the full length of the sequences being compared.

The term "immune response" refers to the action of, for example, lymphocytes, antigen presenting cells, phagocytic cells, granulocytes, and soluble macromolecules produced by the above cells or the liver (including antibodies, cytokines, and complements), this action results in selective damage to, destruction of or elimination from the human body of invading pathogens, pathogen-infected cells or tissues, cancer cells or normal human cells or tissues in the case of autoimmune or pathological inflammation.

The term "signal transduction pathway" or "signal transduction activity" refers to a biochemical causal relationship, usually initiated by protein-protein interactions, such as the binding of a growth factor to a receptor; this relationship results in the transmission of a signal from one part of a cell to another part. Typically, transmission involves specific phosphorylation of one or more tyrosine, serine, or threonine residues on one or more proteins in a cascade of reactions leading to signal transduction. The penultimate process often involves nuclear events, resulting in changes in gene expression.

The terms "activity" or "biological activity", or the terms "biological property" or "biological characteristics" are used interchangeably herein, including but not limited to epitope/antigen affinity and specificity, ability to neutralize or antagonize CLDN-18.2 activity *in vivo* or *in vitro,* IC₅₀, *in vivo* stability of the antibody and immunogenic property of the antibody. Other identifiable biological properties or characteristics of antibodies known in the art include, for example, cross-reactivity (*i.e.*, cross-reactive usually with a non-human homologue of a target peptide, or with other proteins or tissues.), and ability to maintain high expression level of proteins in mammalian cells. The aforementioned properties or characteristics are observed, measured or evaluated using techniques known in the art including, but not limited to, ELISA, FACS or BIACORE plasma resonance analysis, unrestricted *in vitro* or *in vivo* neutralization assays, receptor binding, cytokine or growth factor production and/or secretion, signal transduction and immunohistochemistry of tissue sections of different origins, including human, primate or any other origins.

The term "antibody" refers to any form of antibody that possesses the desired biological activity. Accordingly, it is used in the broadest sense and specifically includes, but is not limited to, monoclonal antibody (including full length monoclonal antibody), polyclonal antibody, multispecific antibody(such as bispecific antibody), humanized antibody, fully human antibody, chimeric antibody and camelized single domain antibody.

The term "isolated antibody" refers to the purified state of a binding compound, and in this context means that the molecule is substantially free of other biomolecules such as nucleic acids, proteins, lipids, sugars or other substances such as cell debris and growth media. The term "isolate (isolated)" does not imply the complete absence of such substances or the absence of water, buffers or salts, unless they are present in an amount that significantly interferes with the experimental or therapeutic use of the binding compound described herein.

The term "monoclonal antibody" refers to an antibody obtained from a substantially homogeneous population of antibody, *i.e*., the individual antibodies making up the population are identical except for possible naturally occurring mutations that may be present in minor amounts. Monoclonal antibodies are highly specific and are directed against a single antigen epitope. In contrast, conventional (polyclonal) antibody preparations typically include a large number of antibodies against (or specific for) different epitopes. The modifier "monoclonal" indicates the characteristic of an antibody obtained from a substantially homogeneous population of antibody and should not be construed as requiring production of the antibody by any particular method.

The term "full length antibody" refers to an immunoglobulin molecule comprising four peptide chains as it occurs in nature: two heavy (H) chains (approximately 50-70 kDa in full length) and two light (L) chains (approximately 25 kDa in full length) connected to each other by disulfide bonds. Each heavy chain consists of a heavy chain variable region (abbreviated herein as VH) and a heavy chain constant region (abbreviated herein as CH). The heavy chain constant region consists of 3 domains (CH1, CH2 and CH3). Each light chain consists of a light chain variable region (abbreviated herein as VL) and a light chain constant region. The light chain constant region consists of one domain CL. The VH and VL regions can be further subdivided into highly variable complementarity determining regions (CDRs) and more conserved regions called framework regions (FRs) spaced by CDRs. Each VH or VL region consists of 3 CDRs and 4 FRs arranged from amino terminus to carboxyl terminus, in the following order: FR1, CDR1, FR2, CDR2, FR3, CDR3, FR4. The variable regions of the heavy and light chains contain binding domains that interact with antigens. The constant region of the antibody can mediate the binding of the immunoglobulin to a host tissue or factor (comprising various cells (e.g., effector cells) of the immune system and the first component of the classical complement system (Clq)).

The term "Fc region" is used to define the C-terminal region of an immunoglobulin heavy chain that contains at least a portion of the constant region. The term includes native sequence Fc regions and variant Fc regions. In one embodiment, the human IgG heavy chain Fc region extends from Cys226 or from Pro230 of the heavy chain to the carboxy-terminus. However, the C-terminal lysine (Lys447) of the Fc region may or may not be present. Unless otherwise indicated herein, the numbering of amino acid residues in the Fc region or constant region is according to the EU numbering system, also known as the EU index, as described in Kabat, E.A. *et al.,* Sequences of Proteins of Immunological Interest, 5th edition, Public Health Service National Institutes of Health, Bethesda, MD (1991), NIH Publication 91-3242.

The term "antigen binding fragment" of an antibody ("parent antibody") includes a fragment or a derivative of an antibody, typically comprising at least a fragment of the antigen binding region or the variable region (for example, one or more CDRs) of the parent antibody, and the fragment or the derivative of the antibody retains at least some of the binding specificity of the parent antibody. Examples of antigen binding fragment include, but are not limited to, Fab, Fab', F(ab')₂ and Fv fragments; diabodies; linear antibodies; single-stranded antibody molecules, such as sc-Fv; and nanobodies and multispecific antibodies formed from antibody fragments. When the binding activity of an antigen is expressed on a molar concentration basis, a binding fragment or a derivative typically retains at least 10% of its antigen binding activity. Preferably the binding fragment or the derivative retains at least 20%, 50%, 70%, 80%, 90%, 95% or 100% or more of the antigen binding affinity of the parent antibody. It is also contemplated that an antigen binding fragment of an antibody may include conservative or non-conservative amino acid substitutions that do not significantly change its biological activity (referred to as "conservative variants" or "functionally conservative variants" of the antibody). The term "binding compound" refers to both an antibody and a binding fragment thereof.

The term "single-chain Fv" or "scFv" antibody refers to an antibody fragment comprising the VH domain and the VL domain of the antibody, wherein these domains are present in a single polypeptide chain. Fv polypeptide generally further comprise a polypeptide linker between the VH domain and the VL domain, which enables the scFv to form the desired structure for antigen binding.

The term "domain antibody" is an immunologically functional immunoglobulin fragment containing only the variable region of a heavy chain or the variable region of a light chain. In certain instances, two or more VH regions are covalently linked with a peptide linker to form a bivalent domain antibody. The two VH regions of the bivalent domain antibody may target the same or different antigens.

The term "bivalent antibody" comprises two antigen binding sites. In some cases, the two binding sites have the same antigen specificity. However, the bivalent antibody can be bispecific.

The term "diabody" refers to a small antibody fragment with two antigen binding sites, the fragment comprises a heavy chain variable domain (VH) linked to a light chain variable domain (VL) in the same polypeptide chain (VH-VL or VL-VH). By using a linker that is too short to allow pairing between the two domains in the same chain, the domain is forced to pair with the complementary domain of another chain to create two antigen binding sites.

The term "chimeric antibody" is an antibody having a variable domain of a first antibody and a constant domain of a second antibody, wherein the first antibody and the second antibody are from different species. Typically, the variable domain is derived from an antibody of a rodent, *etc.* (a "parent antibody"), while the constant domain sequence is derived from a human antibody such that compared with the parental rodent antibody, the obtained chimeric antibody is less likely to induce an adverse immune response in human subjects.

The term "humanized antibody" refers to a form of an antibody that contains sequences from both human and non-human (for example, mouses and rats) antibody. In general, a humanized antibody comprises at least one, usually two, variable domains, wherein all or substantially all of the hypervariable loops correspond to those of a non-human immunoglobulin, and all or substantially all of the framework (FR) regions correspond to those of a human immunoglobulin sequence. A humanized antibody optionally can comprise at least a portion of a human immunoglobulin constant region (Fc).

The term "fully human antibody" refers to an antibody that comprises only human immunoglobulin protein sequences. If the fully human antibody is produced in mice, mouse cells or hybridomas derived from mouse cells, the fully human antibody may contain mouse sugar chains. Likewise, "mouse antibody" refers to an antibody comprising only mouse immunoglobulin sequences. Alternatively, if the fully human antibody is produced in rats, rat cells or hybridomas derived from rat cells, the fully human antibody may contain rat sugar chains. Likewise, "rat antibody" refers to an antibody that comprises only rat immunoglobulin sequences.

An "isotype" antibody refers to the class of antibody (for example, IgM, IgE, and IgG such as IgG1, IgG2 or IgG4) based on the heavy chain constant region genes. The isotype also includes modified forms of one of these classes, wherein modifications have been made to alter Fc function, for example to enhance or attenuate effector function or binding to Fc receptors.

The term "epitope" refers to an antigen region to which an antibody binds. The epitope can be formed from contiguous amino acids or non-contiguous amino acids juxtaposed by the tertiary folding of the protein.

"Affinity" or "binding affinity" refers to the intrinsic binding affinity that reflects the interaction between members of a binding pair. The affinity of a molecule X for its partner Y can generally be represented by the equilibrium dissociation constant (K_{D}), which is ratio of the dissociation rate constant (k_{dis}) to the association rate constant (kₒₙ). Affinity can be measured by common methods known in the art. One specific method for measuring affinity is the ForteBio Kinetic Binding Assay herein.

The term "not binding" to a protein or cell means not binding to the protein or cell, or not binding to the protein or cell with high affinity, *i.e.,* the K_{D} for binding to the protein or cell is 1.0 × 10⁻⁶ M or more, more preferably 1.0 × 10⁻⁵ M or more, more preferably 1.0 × 10⁻⁴ M or more, 1.0 × 10⁻³ M or more, and more preferably 1.0 × 10⁻² M or more.

The term "high affinity" for an IgG antibody means a K_{D} for a antigen of 1.0 × 10⁻⁶ M or less, preferably 5.0 × 10⁻⁸ M or less, more preferably 1.0 × 10⁻⁸ M or less, 5.0 × 10⁻⁹M or less, and more preferably 1.0 × 10⁻⁹M or less. "High affinity" binding may vary for other antibody subtypes. For example, "high affinity" binding of the IgM subtype means a K_{D} of 10⁻⁶ M or less, preferably 10⁻⁷ M or less, and more preferably 10⁻⁸ M or less.

The terms "antibody-dependent cellular cytotoxicity", "antibody-dependent cell-mediated cytotoxicity" or "ADCC" refer to cell-mediated immune defense in which immune system effector cells actively lyse target cells, such as cancer cells, which has their cell membrane surface antigens bound by antibodies, such as Claudin18.2 antibody.

The term "complement dependent cytotoxicity" or "CDC" refers to the effector functions of IgG and IgM antibodies which, when bound to surface antigens, initiate the canonical complement pathway, including formation of a membrane attack complex and lysis of target cells. The antibody of the present invention, when bound to Claudin 18.2, induces CDC on cancer cells.

The term "nucleic acid" or "polynucleotide" refers to deoxyribonucleic acid (DNA) or ribonucleic acid (RNA) and polymers thereof in single- or double-stranded form. Unless expressly limited, the term includes nucleic acids containing analogs of known natural nucleotides and having similar binding properties to the reference nucleic acid and metabolized in a manner similar to naturally occurring nucleotides (see U.S. pat. No.8,278,036 to Kariko *et al.,* which discloses an mRNA molecule in which uridine is replaced by pseudouridine, a method for synthesizing the mRNA molecule, and a method for delivering therapeutic proteins *in vivo*)*.* Unless otherwise indicated, a particular nucleic acid sequence also implicitly encompasses conservatively modified variants thereof (for example, degenerate codon substitutions), alleles, orthologs, SNPs, and complementary sequences as well as the sequences explicitly indicated. Specifically, degenerate codon substitutions can be achieved by generating sequences in which the third positions of one or more selected (or all) codons are replaced with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" means any recombinant polynucleotide molecule (such as a plasmid, cosmid, virus, autonomously replicating polynucleotide molecule, bacteriophage, or linear or circular single- or double-stranded DNA or RNA polynucleotide molecule), which is derived from any source, capable of integrating with the genome or replicating autonomously, constitutes a polynucleotide molecule in which one or more polynucleotide molecules have been functionally linked (*i.e.,* operably linked). A recombinant construct typically comprises a polynucleotide of the present invention operably linked to a transcription initiation regulatory sequence that direct transcription of the polynucleotide in a host cell. Both heterologous and non-heterologous (*i.e.,* endogenous) promoters can be used to direct expression of the nucleic acids of the present invention.

"Vector" refers to any recombinant polynucleotide construct that can be used for the purpose of transformation (*i*.e., introduction of a heterologous DNA into a host cell). One type of vector is a "plasmid", which refers to a circular double-stranded DNA loop into which additional DNA segments can be ligated. Another type of vector is a viral vector, and additional DNA segments can be ligated into the viral genome. Certain vectors are capable of autonomous replication in a host cell into which they are introduced (for example, bacterial vectors having a bacterial origin of replication and episomal mammalian vectors). After introduction into a host cell, other vectors (for example, non-episomal mammalian vectors) integrate into the genome of the host cell and thus replicate along with the genome of the host cell. In addition, certain vectors are capable of directing the expression of operably linked genes. Such vector is referred to as "expression vector" herein.

The term "expression vector" as used herein refers to a nucleic acid molecule capable of replicating and expressing a gene of interest upon transformation, transfection or transduction into a host cell. The expression vector contains one or more phenotypic selectable markers and an origin of replication to ensure maintenance of the vector and to provide amplification in the host if desired.

"Activation", "stimulation" and "treatment" for a cell or receptor can have the same meaning, for example, the cell or receptor is activated, stimulated or treated with a ligand, unless the context dictates otherwise or explicitly. "Ligand" includes natural and synthetic ligands such as cytokines, cytokine variants, analogs, muteins, and binding compounds derived from antibodies. "Ligand" also includes small molecules, such as peptidomimetics of cytokines and peptidomimetics of antibodies. "Activation" can refer to cellular activation regulated by internal mechanisms as well as external or environmental factors. "Response/reaction", such as response of a cell, tissue, organ, or organism, includes changes in biochemical or physiological behaviors (such as concentration, density, adhesion or migration, gene expression rate, or differentiation status within a biological compartment), where the changes are related to activation, stimulation or treatment, or to internal mechanisms such as genetic programming.

As used herein, the term "treat" or "treatment" of any disease or condition refers in one embodiment to ameliorating the disease or condition (*i.e*., slowing or arresting or reducing the progression of or at least one clinical symptom of the disease). In another embodiment, "treat" or "treatment" refers to alleviating or improving at least one physical parameter, including those physical parameters that may not be discernible by the patient. In another embodiment, "treat" or "treatment" refers to modulating a disease or condition physically (for example, stabilization of discernible symptoms), physiologically (for example, stabilization of physical parameters), or both. Unless explicitly described herein, methods for assessing treatment and/or prevention of a disease are generally known in the art.

"Subject" includes any human or non-human animal. The term "non-human animal" includes all vertebrates, for example, mammals and non-mammals, such as non-human primates, sheep, dogs, cats, horses, cows, chickens, amphibians, reptiles, and the like. As used herein, the term "cyno" or "cynomolgus monkey" refers to cynomolgus monkeys.

Administration "in combination with" one or more other therapeutic agents includes both simultaneous (co)administration and sequential administration in any order.

"Therapeutically effective amount", "therapeutically effective dose" and "effective amount" refers to the amount of the CLDN-18.2 antibodies or the antigen binding fragments of the present invention that can effectively prevent or improve the symptoms of one or more diseases or conditions or the development of such diseases or conditions when administered to cells, tissues or subjects alone or in combination with other therapeutic agents. A therapeutically effective dose also refers to an amount of an antibody or an antigen binding fragment thereof sufficient to result in an amelioration of symptoms, for example, an amount that treats, cures, prevents or ameliorates an associated medical condition or increases the rate of treatment, cure, prevention or amelioration of such a condition. When the active ingredient is administered alone to a subject, the therapeutically effective dose refers to that ingredient only. When administered in combination, the therapeutically effective dose refers to the combined amount of the active ingredients which produces the therapeutic effect, whether in combination, sequentially or simultaneously. An effective amount of a therapeutic agent will result in an improvement in diagnostic criteria or parameters of at least 10%; usually at least 20%; preferably at least about 30%; more preferably at least 40%, and most preferably at least 50%.

"Cancer" and "cancerous" refer to or describe the physiological illness in mammals that is often characterized by unregulated cell growth. Included in this definition are benign and malignant cancers as well as dormant tumors or micrometastases. Examples of cancers include, but are not limited to, carcinoma, lymphoma, blastoma, sarcoma, and leukemia. More specific examples of such cancers include squamous cell carcinoma, lung cancer (including small cell lung cancer, non-small cell lung cancer, adenocarcinoma of the lung, and squamous cell carcinoma of the lung), peritoneal carcinoma, hepatocellular carcinoma, cancer of the stomach or gastric cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial cancer or uterine cancer, salivary gland cancer, renal cancer or cancer of the kidney, hepatic cancer, prostate cancer, vulvar cancer, thyroid cancer, cancer of the liver, and various types of head and neck cancer, and B-cell lymphoma (including low-grade/follicular non-Hodgkin King's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphoma, and Waldenstrom's macroglobulinemia), chronic lymphocytic leukemia (CLL), acute lymphoblastic leukemia (ALL), hairy cell leukemia, chronic myeloblastic leukemia, and post-transplant lymphoproliferative disorder (PTLD), as well as abnormal vascular proliferation associated with phakomatoses, edema (such as those associated with brain tumors) and Meigs's syndrome.

### Anti-CLDN-18.2 antibody

In one aspect, the present invention provides an anti-CLDN-18.2 antibody or an antigen binding fragment thereof. The term "anti-CLDN-18.2 antibody", "anti-CLDN-18.2", "CLDN-18.2 antibody" or "antibody that binds to CLDN-18.2" means that it can bind to CLDN-18.2 protein or fragment thereof with sufficient affinity so that the antibody can be used as a diagnostic and/or therapeutic agent targeting CLDN-18.2.

The antibody of the present invention can be produced using any suitable method for producing antibodies. Any suitable form of CLDN-18.2 can be used as an immunogen (antigen) for antibody production. By way of example and not limitation, any CLDN-18.2 variant or fragment thereof can be used as an immunogen. In some embodiments, hybridoma cells producing murine monoclonal anti-human CLDN-18.2 antibody can be produced by methods known in the art. The antibody derived from rodents (for example, mice) may cause unwanted antibody immunogenicity when used *in vivo* as a therapeutic drug, and repeated use leads to the immune response of the human body against therapeutic antibodies, and such immune response at least leads to the loss of therapeutic efficacy, and in severe cases leads to potentially fatal allergic reactions. One approach to reducing the immunogenicity of rodent antibodies includes the generation of chimeric antibodies, in which mouse variable regions are fused to human constant regions (Liu et al. (1987) Proc. Natl. Acad. Sci. USA 84:3439- 43). However, retention of intact rodent variable regions in the chimeric antibodies may still cause deleterious immunogenicity in patients. Grafting of complementarity determining region (CDR) loops of rodent variable domains onto human frameworks (*i.e*. humanization) has been used to further minimize rodent sequences (Jones et al. (1986) Nature 321:522; Verhoeyen et al. (1988) Science 239:1534).

In some embodiments, the chimeric or humanized antibody of the present invention can be prepared based on the sequence of the prepared murine monoclonal hybridoma antibody. DNA coding heavy and light chain immunoglobulins can be obtained from murine hybridomas of interest and engineered to contain non-murine (for example, human) immunoglobulin sequences using standard molecular biology techniques.

In some embodiments, the chimeric CLDN-18.2 antibody of the present invention can be prepared by effectively linking the variable regions of immunoglobulin heavy chain and light chain from hybridoma with the constant region of human IgG (see, for example, U.S. Pat. No.4,816,567 belonging to Cabilly et al.) by methods known in the art to obtain a chimeric heavy chain and a chimeric light chain. In some embodiments, the constant region comprised in the chimeric antibody of the present invention can be selected from any human IgG subtype, such as IgG1, IgG2, IgG3, IgG4, preferably IgG4.

In some embodiments, the chimeric CLDN-18.2 antibody of the present invention can be obtained by transfecting expression cells with an expression plasmid of the chimeric light chain and the chimeric heavy chain in a manner of "mix and match", and the above binding assay and other conventional binding assays (for example, ELISA) can be used to determine the CLDN-18.2 binding of such "mixed and matched" antibody.

The precise amino acid sequence boundaries of the variable region CDRs of the antibody of the present invention can be determined using any of a number of well-known schemes, including Chothia based on the three-dimensional structure of an antibody and the topology of a CDR loop (Chothia et al. (1989) Nature 342:877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (1999 Nucleic Acids Research, 27, 209-212), and the North CDR definition based on affinity propagation clustering using a large number of crystal structures. The boundaries of the CDRs of the antibody of the present invention can be determined by a person skilled in the art according to any scheme in the art (e.g., different assignment systems or combinations).

It should be noted that the boundaries of the CDRs of the variable regions of the same antibody obtained based on different assignment systems may be different. That is, the CDR sequences of the variable regions of the same antibody defined under different assignment systems are different. Therefore, when it comes to defining an antibody with specific CDR sequences defined in the present invention, the scope of the antibody also encompasses such an antibody whose variable region sequences comprise the specific CDR sequences but whose claimed CDR boundaries are different from the specific CDR boundaries defined by the present invention due to the application of a different scheme (for example, different assignment systems or combinations).

Antibodies with different specificities (*i.e.,* different binding sites for different antigens) have different CDRs. However, although CDRs vary from antibody to antibody, only a limited number of amino acid positions within a CDR are directly involved in antigen binding. Using at least two of the methods of Kabat, Chothia, AbM, Contact and North, a minimum overlapping region can be determined, thereby providing a "minimum binding unit" for antigen binding. The minimal binding unit may be a subpart of a CDR. As will be apparent to those skilled in the art, the residues of the remainder of the CDR sequences can be determined from the structure and protein folding of the antibody. Accordingly, the present invention also contemplates variants of any of the CDRs presented herein. For example, in a variant of a CDR, the amino acid residues of the minimum binding unit can remain unchanged, while the remaining CDR residues defined according to Kabat or Chothia can be replaced by conserved amino acid residues.

For the humanized antibody of the present invention, the murine CDR regions can be inserted into the human germline framework regions using methods known in the art. See U.S. Patent No. 5,225,539 to Winter et al. and U.S. Patent No. 5,530,101; 5,585,089; 5,693,762 and 6,180,370 to Queen et al.

In some embodiments, amino acid changes include amino acid deletions, insertions or substitutions. In some embodiments, the anti-CLDN-18.2 antibodies or the antigen binding fragments thereof of the present invention include those antibodies that have amino acid sequences have been mutated by amino acid deletion, insertion or substitution, but still have at least about 90%, 91%, 92%, 93%, 94%, 95%, 96%, 97%, 98%, 99% or 100% identity to the above antibodies (especially in the CDR regions depicted in the above sequences). In some embodiments, the antibody of the present invention has no more than 1, 2, 3, 4 or 5 amino acid mutations in the CDR region by amino acid deletion, insertion or substitution when compared to the CDR region depicted in the specific sequence.

In some embodiments, the polynucleotide coding the antibody of the present invention include a polynucleotide that have been mutated by nucleotide deletion, insertion, or substitution but still have at least about 60, 70, 80, 90, 95, or 100% identity to the coding region corresponding to the CDR depicted in the sequence described above.

In some embodiments, one or more amino acid modifications can be introduced into the Fc region of the antibody provided herein to generate a Fc region variant. The Fc region variant may comprise human Fc region sequences (for example, human IgG1, IgG2, IgG3 or IgG4 Fc regions) comprising amino acid modifications (for example, substitutions) at one or more amino acid positions.

In some embodiments, it may be desirable to generate a cysteine-engineered antibody, such as "thioMAb," in which one or more residues of the antibody are replaced with cysteine residues.

In some embodiments, an antibody can be altered to increase or decrease its degree of glycosylation and/or alter its glycosylation pattern. Addition or deletion of glycosylation sites to an antibody is conveniently accomplished by altering the amino acid sequence to create or remove one or more glycosylation sites. For example, one or more amino acid substitutions can be made to eliminate one or more glycosylation sites, thereby eliminating glycosylation at that site. The antibody can be prepared with altered types of glycosylation, for example, a hypofucosylated antibody with reduced amount of fucosyl residues or an afucosylated antibody or an antibody with increased bisected GlcNac structures. Such altered glycosylation patterns have been shown to increase the ADCC ability of an antibody.

In some preferred embodiments, the present invention provides an antibody that is hypofucosylated or afucosylated such that the binding affinity of the antibody to receptors expressed on effector cells can be significantly increased, thereby resulting in the antibody having enhanced antibody-dependent cell-mediated cytotoxicity (ADCC) activity. The average value of fucose in the sugar chain at Asn 297 can be calculated relative to the sum of all sugar structures (such as complex, hybrid and high mannose structures) linked to Asn297 by MALDI-TOF mass spectrometry, thereby determining the amount of fucose, for example as described in WO 2008/077546. Asn297 refers to the aspartic acid residue located at approximately position 297 (EU numbering of Fc region residues) in the Fc region; however, due to minor sequence variations in the antibody, Asn297 may also be located approximately ± 3 amino acid positions upstream or downstream of position 297, *i.e.,* between position 294 and position 300. See, for example, US 2003/0157108; US 2004/0093621. Such antibody variants can be produced in cell lines capable of producing defucosylated or hypofucosylated antibodies. Examples of such cells include Lecl3 CHO cells deficient in protein fucosylation (Ripka, J. et al., Arch. Biochem. Biophys. 249 (1986): 533-545; US 2003/0157108). In some embodiments, fucose residues of the antibody are cleaved using fucosidase. In some embodiments, glycoform modulators are used to manipulate the fucose residues of the antibody. The glycoform modulators may be CDFS01, which is commercially available from Shanghai OPM Biosciences Co., Ltd. Defucosylation can be performed by conventional methods known in the art.

The level of fucosylation of an antibody can be structurally defined. As used herein, "non-fucosylation" or "afucosylation" means that in the antibody, the content of fucose is less than 5%, such as about 0%, less than 1%, less than 2%, less than 3%, and less than 4%. The term "hypofucosylation" means that in the antibody, the content of fucose is about greater than or equal to 5% and less than 30%; for example, the content of fucose in the antibody is about 5%-10%, 10%-15%, 15%-20%, 20%-25%, 25%-30%, 10%-20%, 20%-30 %, and 10%-30%. The term "hypofucosylation or afucosylation" means that the content of fucose in the antibody is less than 30%.

In some embodiments, the antibody provided herein can be further modified to contain other non-protein moieties known and readily available in the art. Moieties suitable for antibody derivatization include, but are not limited to, water soluble polymers. Non-limiting examples of water soluble polymers include, but are not limited to, polyethylene glycol (PEG), ethylene glycol/propylene glycol copolymer, carboxymethylcellulose, dextran, polyvinyl alcohol, polyvinylpyrrolidone, poly-1,3-dialkyl, poly-1,3,6-trialkyl, ethylene/maleic anhydride copolymer, polyamino acid (homopolymer or random copolymer), and dextran or poly(n-vinyl pyrrolidone) polyethylene glycol, propylene glycol homopolymer, polypropylene oxide/ethylene oxide copolymer, polyoxyethylated polyols (such as glycerin), polyvinyl alcohol, and mixtures thereof.

### Antibody expression

In yet another aspect, the present invention provides a polynucleotide coding the anti-CLDN-18.2 antibody or the antigen binding fragment thereof as described herein. The polynucleotide may comprise a polynucleotide coding the amino acid sequence of the light chain variable region and/or the heavy chain variable region of the antibody, or comprise a polynucleotide coding the amino acid sequence of the light chain and/or the heavy chain of the antibody.

In yet another aspect, the present invention provides an expression vector comprising the polynucleotide as described herein, preferably, the vector is a eukaryotic expression vector. In some embodiments, the polynucleotide as described herein is contained in one or more expression vectors.

In yet another aspect, the present invention provides a host cell comprising the polynucleotide as described herein or the expression vector as described herein, preferably, the host cell is a eukaryotic cell, more preferably a mammalian cell.

In yet another aspect, the present invention provides a method for preparing the anti-CLDN-18.2 antibody or the antigen binding fragment thereof as described herein, the method comprises expressing the antibody or the antigen binding fragment thereof in the host cell as described herein under conditions suitable for expression of the antibody or the antigen binding fragment thereof, and recovering the expressed antibody or the antigen binding fragment thereof from the host cell.

The prensent invention provides mammalian host cells for expressing the recombinant antibody of the present invention, including the many immortalized cell lines available from the American Type Culture Collection (ATCC). These include, *inter alia,* Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells and many others cell line. The mammalian host cells include human, mouse, rat, dog, monkey, pig, goat, bovine, horse and hamster cells. Particularly preferred cell lines are selected by determining which cell lines have high expression levels.

In one embodiment, the present invention provides a method for preparing an anti-CLDN-18.2 antibody, wherein the method comprises that an expression vector is introduced into a mammalian host cell, and the host cell is cultured for a sufficient period of time to allow the antibody to be expressed in the host cell, or more preferably to allow the antibody to be secreted into the culture medium where the host cell grows, thereby producing the antibody. The antibody can be recovered from the culture medium using standard protein purification methods.

It is likely that antibodies expressed by different cell lines or expressed in transgenic animals have different glycosylation from each other. However, all the antibodies coded by the nucleic acid molecules provided herein or comprising the amino acid sequences provided herein are part of the present invention, regardless of the glycosylation of the antibodies. Also, in certain embodiments, non-fucosylated antibodies are advantageous because they generally have stronger efficacy than their fucosylated counterparts *in vitro* and *in vivo* and are less likely to be immunogenic, because their sugar structure is a normal component of natural human serum IgG.

### Drug conjugate

The present invention provides an anti-CLDN-18.2 antibody-drug conjugate (ADC) with a structure shown in formula (I-1), or a pharmaceutically acceptable salt thereof:

Ab-(L-D)m formula (I-1)

wherein,
L is a linker unit;
D is a small molecule drug with cytotoxicity;
m represents the average number of L-D units conjugated to Ab, and m is selected from 1-8, preferably 2-7, more preferably 2-5;
Ab is an anti-CLDN-18.2 antibody or an antigen-binding fragment thereof, which comprises
   (I) a heavy chain variable region comprising HCDR1 and HCDR2 with amino acid sequences as shown in SEQ ID NO: 1 and SEQ ID NO: 72, respectively, and HCDR3 as shown in SEQ ID NO: 73 or 3; and a light chain variable region comprising LCDR1, LCDR2 and LCDR3 with amino acid sequences as shown in SEQ ID NO: 7, SEQ ID NO: 8 and SEQ ID NO: 9, respectively; or
   (II) a heavy chain variable region comprising HCDR1, HCDR2 and HCDR3 with amino acid sequences as shown in SEQ ID NO: 1, SEQ ID NO: 41 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
   (III) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
   (IV) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 19, SEQ ID NO: 20 and SEQ ID NO: 21, respectively; or
   (V) a heavy chain variable region comprising HCDR1 and HCDR2 with amino acid sequences as shown in SEQ ID NO: 28 and SEQ ID NO: 74, respectively, and HCDR3 as shown in SEQ ID NO: 30 or 75; and a light chain variable region comprising LCDR1, LCDR2 and LCDR3 with amino acid sequences as shown in SEQ ID NO: 13, SEQ ID NO: 14 and SEQ ID NO: 15, respectively.

In some embodiments, the anti-CLDN-18.2 antibody-drug conjugate of the present invention is represented by the following formula: 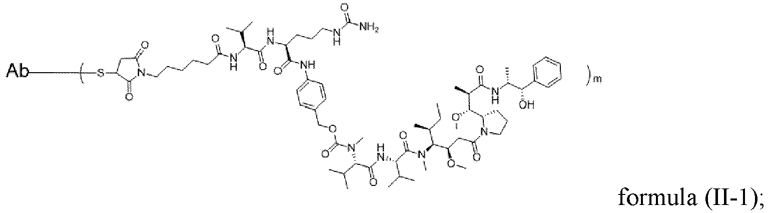 or 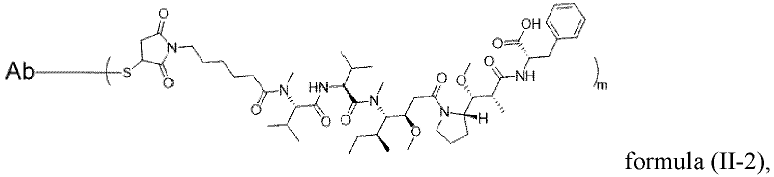 wherein m and Ab are as defined herein.

In some embodiments, the anti-CLDN-18.2 antibody or antigen-binding fragment thereof of the present invention comprises:
(I) a heavy chain variable region with an amino acid sequence as shown in SEQ ID NO: 55, 57, 82 or 83, and a light chain variable region with an amino acid sequence as shown in SEQ ID NO:56; or
(II) a heavy chain variable region with an amino acid sequence as shown in SEQ ID NO: 60, 76 or 78, and a light chain variable region with an amino acid sequence as shown in SEQ ID NO:61; or
(III) a heavy chain variable region with an amino acid sequence as shown in SEQ ID NO: 76, and a light chain variable region with an amino acid sequence as shown in SEQ ID NO: 77; or
(IV) a heavy chain variable region with an amino acid sequence as shown in SEQ ID NO: 79; and a light chain variable region with an amino acid sequence as shown in SEQ ID NO:80; or
(V) a heavy chain variable region with an amino acid sequence as shown in SEQ ID NO: 55 , and a light chain variable region with an amino acid sequence as shown in SEQ ID NO: 81.

In some embodiments, the anti-CLDN-18.2 antibody or antigen-binding fragment thereof of the present invention comprises:
(1) a heavy chain with an amino acid sequence as shown in SEQ ID NO: 64 , and a light chain with an amino acid sequence as shown in SEQ ID NO: 65; or
(2) a heavy chain with an amino acid sequence as shown in SEQ ID NO: 68 , and a light chain with an amino acid sequence as shown in SEQ ID NO: 69; or
(3) a heavy chain with an amino acid sequence as shown in SEQ ID NO: 84 , and a light chain with an amino acid sequence as shown in SEQ ID NO: 85; or
(4) a heavy chain with an amino acid sequence as shown in SEQ ID NO: 86 , and a light chain with an amino acid sequence as shown in SEQ ID NO: 87; or
(5) a heavy chain with an amino acid sequence as shown in SEQ ID NO: 88 , and a light chain with an amino acid sequence as shown in SEQ ID NO: 89; or
(6) a heavy chain with an amino acid sequence as shown in SEQ ID NO: 90 , and a light chain with an amino acid sequence as shown in SEQ ID NO: 91; or
(7) a heavy chain with an amino acid sequence as shown in SEQ ID NO: 92 , and a light chain with an amino acid sequence as shown in SEQ ID NO: 93; or
(8) a heavy chain with an amino acid sequence as shown in SEQ ID NO: 94 , and a light chain with an amino acid sequence as shown in SEQ ID NO: 95; or
(9) a heavy chain with an amino acid sequence as shown in SEQ ID NO: 96 , and a light chain with an amino acid sequence as shown in SEQ ID NO: 97; or
(10) a heavy chain with an amino acid sequence as shown in SEQ ID NO: 98 , and a light chain with an amino acid sequence as shown in SEQ ID NO: 99; or
(11) a heavy chain with an amino acid sequence as shown in SEQ ID NO: 100 , and a light chain with an amino acid sequence as shown in SEQ ID NO: 101.

### Pharmaceutical composition and pharmaceutical preparation

In yet another aspect, the present invention provides a pharmaceutical composition comprising the anti-CLDN-18.2 antibody-drug conjugate or the pharmaceutically acceptable salt thereof as described herein, and a pharmaceutically acceptable carrier or excipient.

It should be understood that the anti-CLDN-18.2 antibody-drug conjugate or the pharmaceutically acceptable salt thereof, or the pharmaceutical composition thereof provided in the present invention can be administered in combination with appropriate carriers, excipients and other agents in the preparation, so as to provide improved transfer, delivery, tolerance and the like.

The term "pharmaceutical composition" refers to a preparation that allows the active ingredients contained therein to be present in a biologically effective form and does not contain additional ingredients that would be unacceptably toxic to the subject to which the preparation is administered.

The anti-CLDN-18.2 antibody-drug conjugate or the pharmaceutically acceptable salt thereof of the present invention having the desired purity can be mixed with one or more optional pharmaceutical excipients (Remington's Pharmaceutical Sciences, 16th edition, Osol, A. ed. (1980)) to prepare the pharmaceutical preparation comprising the anti-CLDN-18.2 antibody described herein, preferably in the form of an aqueous solution or a lyophilized preparation.

The pharmaceutical composition or preparation of the present invention may also comprise one or more other active ingredients as required for the particular indication being treated, preferably those active ingredients with complementary activities that do not adversely affect each other. In some embodiments, other active ingredients are chemotherapeutic agents, immune checkpoint inhibitors, growth inhibitors, antibiotics, or various known anti-tumor or anti-cancer agents, and the active ingredients are present in a suitable combination in an amount effective for the intended use. In some embodiments, the pharmaceutical composition of the present invention further comprises a composition of a polynucleotide coding the anti-CLDN-18.2 antibody.

In yet another aspect, the present invention provides a pharmaceutical combination comprising the antibody-drug conjugate or pharmaceutically acceptable salt thereof as described herein or the pharmaceutical composition as described herein, and one or more additional therapeutic agents.

In yet another aspect, the present invention provides a kit comprising the antibody-drug conjugate or pharmaceutically acceptable salt thereof as described herein or the pharmaceutical composition as described herein, preferably further comprising a drug delivery device.

### Medicinal use

In yet another aspect, the present invention provides use of the antibody-drug conjugate, the pharmaceutically acceptable salt thereof as described herein, or the pharmaceutical composition as described herein in the preparation of a medicament for the treatment and/or prevention of a disease or condition mediated by CLDN-18.2; preferably, the disease or condition is a cancer.

In yet another aspect, the present invention provides the antibody-drug conjugate or pharmaceutically acceptable salt thereof as described herein, or the pharmaceutical composition as described herein, for use in the treatment and/or prevention of a disease or condition mediated by CLDN-18.2; preferably, the disease or condition is a cancer.

In yet another aspect, the present invention provides a method of treating and/or preventing a disease or condition mediated by CLDN-18.2, comprising administering the antibody-drug conjugate or pharmaceutically acceptable salt thereof as described herein or the pharmaceutical composition as described herein to a subject in need thereof, the disease or condition is a cancer.

In some embodiments, the cancer is selected from gastric cancer, esophageal cancer, gastroesophageal cancer, pancreatic cancer, cholangiocarcinoma, lung cancer, ovarian cancer, colon cancer, liver cancer, head and neck cancer, gallbladder cancer, intestinal cancer, and bladder cancer.

In some embodiments, modes of administration of the present invention include, but are not limited to, oral, intravenous, subcutaneous, intramuscular, intraarterial, intraarticular (for example, in arthritic joints), via inhalation, aerosol delivery, or intratumoral administration and the like.

In some embodiments, the present invention provides administration in combination with a therapeutically effective amount of one or more therapies (for example, treatment modalities and/or other therapeutic agents) to a subject. In some embodiments, the therapies include surgery and/or radiation therapy.

In some embodiments, the method or use provided in the present invention further comprises administering one or more therapies (for example, treatment modalities and/or other therapeutic agents) to an individual. The antibody-drug conjugate or pharmaceutically acceptable salt thereof of the present invention can be used alone or in combination with other therapeutic agents in the therapy. For example, it can be co-administered with at least one additional therapeutic agent.

In one embodiment, the method for treating cancer diseases of the present invention further comprises administering an agent for stabilizing or increasing the expression of CLDN-18.2. Expression of CLDN-18.2 is preferably on the cell surface of cancer cells. The agent for stabilizing or increasing the expression of CLDN-18.2 can be oxaliplatin and/or 5-FU.

The present invention includes all combinations of the particular embodiments described. Further embodiments and the full scope of applicability of the present invention will become apparent from the detailed description provided hereinafter. However, it should be understood that the detailed description and specific examples, while indicating preferred embodiments of the present invention, are given by way of illustration only, since various changes and modifications within the spirit and scope of the present invention will become apparent to those skilled in the art from this detailed description. All publications, patents, and patent applications cited herein, including quotations, are incorporated herein by reference in their entirety for all purposes.

### Examples

The following examples are provided to demonstrate and further explain some preferred embodiments and aspects of the present invention and should not be construed as limiting the scope thereof.

### Example 1. Anti-CLDN-18.2 antibody

The anti-CLDN-18.2 antibody or antigen-binding fragment thereof of the present invention includes any anti-CLDN-18.2 antibody or antigen-binding fragment thereof described in the application number PCT/CN2021/106125. The content disclosed in this application is incorporated herein by reference in its entirety. In some embodiments, the sequences of the anti-CLDN-18.2 antibodies used in the drug-conjugates and compositions of the present invention are as shown in Tables 1 and 2 below.

**Table 1. Humanized anti-CLDN-18.2 antibody variable region and CDR sequences (KABAT scheme)**

| Humanized antibody | JS012-Chi9-hu7 | JS012-Chi9-hu7-v2 | JS012-Chi2-hu2-v3-2/ JS012-Chi2-hu2-v3-2a |
|---|---|---|---|
| HCDR1 | SEQ ID NO: 10 | SEQ ID NO:10 | SEQ ID NO: 1 |
| HCDR2 | SEQ ID NO:40 | SEQ ID NO:40 | SEQ ID NO:41 |
| HCDR3 | SEQ ID NO:12 | SEQ ID NO:12 | SEQ ID NO:3 |
| LCDR1 | SEQ ID NO:13 | SEQ ID NO:13 | SEQ ID NO:7 |
| LCDR2 | SEQ ID NO: 14 | SEQ ID NO:14 | SEQ ID NO:8 |
| LCDR3 | SEQ ID NO: 15 | SEQ ID NO:15 | SEQ ID NO:9 |
| VH | SEQ ID NO:55 | SEQ ID NO:57 | SEQ ID NO:60 |
| VL | SEQ ID NO:56 | SEQ ID NO:56 | SEQ ID NO:61 |
| HC | SEQ ID NO:68 | SEQ ID NO:96 | SEQ ID NO:64 |
| LC | SEQ ID NO:69 | SEQ ID NO:97 | SEQ ID NO:65 |

**Table 2. Humanized anti-CLDN-18.2 antibody variable region and CDR sequences (KABAT scheme)**

| Humanized Antibody | JS012-chi2-hu27 | JS012-chi2-hu29 | JS012-chi2-hu10 V2 | JS012-chi9-hu2 | JS012-chi9-hu8 V2 | JS012-chi10-hu9 | JS012-chi27-hu36 | JS012-Chi27-hu3-v2 |
|---|---|---|---|---|---|---|---|---|
| HCDR1 | SEQ ID NO:1 | SEQ ID NO:1 | SEQ ID NO:1 | SEQ ID NO:10 | SEQ ID NO:10 | SEQ ID NO:10 | SEQ ID NO:28 | SEQ ID NO:28 |
| HCDR2 | SEQ ID NO:72 | SEQ ID NO:72 | SEQ ID NO:72 | SEQ ID NO:40 | SEQ ID NO:40 | SEQ ID NO:40 | SEQ ID NO:74 | SEQ ID NO:29 |
| HCDR3 | SEQ ID NO:73 | SEQ ID NO:73 | SEQ ID NO:3 | SEQ ID NO:12 | SEQ ID NO:12 | SEQ ID NO:12 | SEQ ID NO:75 | SEQ ID NO:30 |
| LCDR1 | SEQ ID NO:7 | SEQ ID NO:7 | SEQ ID NO:7 | SEQ ID NO:13 | SEQ ID NO:13 | SEQ ID NO:19 | SEQ ID NO:13 | SEQ ID NO:13 |
| LCDR2 | SEQ ID NO:8 | SEQ ID NO:8 | SEQ ID NO:8 | SEQ ID NO:14 | SEQ ID NO:14 | SEQ ID NO:20 | SEQ ID NO:14 | SEQ ID NO:14 |
| LCDR3 | SEQ ID NO:9 | SEQ ID NO:9 | SEQ ID NO:9 | SEQ ID NO:15 | SEQ ID NO:15 | SEQ ID NO:21 | SEQ ID NO:15 | SEQ ID NO:15 |
| VH | SEQ ID NO:76 | SEQ ID NO:76 | SEQ ID NO:78 | SEQ ID NO:79 | SEQ ID NO:57 | SEQ ID NO:55 | SEQ ID NO:82 | SEQ ID NO:58 |
| VL | SEQ ID NO:61 | SEQ ID NO:77 | SEQ ID NO:61 | SEQ ID NO:80 | SEQ ID NO:56 | SEQ ID NO:81 | SEQ ID NO:56 | SEQ ID NO:56 |
| HC | SEQ ID NO:84 | SEQ ID NO:86 | SEQ ID NO:88 | SEQ ID NO:90 | SEQ ID NO:92 | SEQ ID NO:94 | SEQ ID NO:98 | SEQ ID NO:102 |
| LC | SEQ ID NO:85 | SEQ ID NO:87 | SEQ ID NO:89 | SEQ ID NO:91 | SEQ ID NO:93 | SEQ ID NO:95 | SEQ ID NO:99 | SEQ ID NO:101 |

### Example 2. Preparation of anti-CLDN-18.2 antibody-drug conjugate (ADC1)

### 2.1 Anti-CLDN-18.2 antibody was coupled to the linker-cytotoxin MC-VC-PAB-MMAE to prepare the conjugate

Drug source: vc-MMAE, namely MC-VC-PAB-MMAE (linker-cytotoxin, CAS number: 646502-53-6), was purchased from Shanghai Haoyuan Chemexpress Co., Ltd.

The anti-CLDN-18.2 antibody was coupled to the linker-cytotoxin MC-VC-PAB-MMAE to prepare the conjugate as follows:
Step 1: 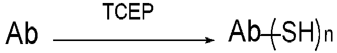
Step 2:

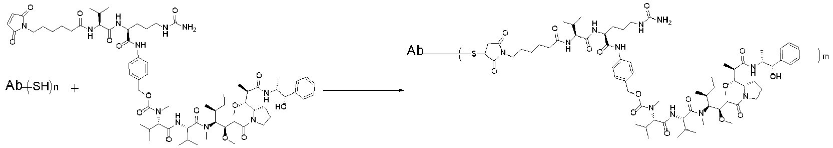

### Preparation of ADC-3-MMAE (JS107-3-MMAE)

Step 1: The antibody JS012-Chi9-hu7 (1.0 eq.) was exchanged into a buffer (pH 7.0, 50 mM PBS/2 mM EDTA), and TCEP (tris(2-carbonylethyl)phosphorus, 2.9 eq.) was added to the resulting exchanged solution, with the temperature controlled at 25°C. The mixture was reacted for 2 h under stirring to afford a solution containing a reduced antibody. Step 2: 10% DMA (N,N-dimethylacetamide) and vc-MMAE (7.0 eq.) were added to the solution containing the reduced antibody, with the temperature controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-3-MMAE of the present invention was obtained, and stored at -60 to -90°C, wherein m = 2-5, and n = 2-5. ADC-3-MMAE was detected by reversed-phase high-performance liquid chromatography, and the ratio of ADC toxin to antibody DAR = 3.97. The SEC-HPLC (size exclusion chromatography) purity of ADC-3-MMAE was 98.9%.

### Preparation of ADC-4-MMAE (JS107-4-MMAE)

Step 1: The antibody JS012-chi2-hu2-v3-2 (1.0 eq.) was exchanged into the buffer (pH 7.0, 50 mM PB/2 mM EDTA), and TCEP (2.9 eq.) was added to the resulting solution, the temperature was controlled at 25°C, the mixture was reacted for 2 h under stirring to afford a solution containing reduced antibody. Step 2: 10% DMA and vc-MMAE (7.0 eq.) were added to the solution containing reduced antibody, the temperature was controlled at 25°C, and the mixture was reacted for 2 h under stirring. The reaction liquid was exchanged into a buffer solution (50 mM PBS/150 mMNaCl/pH 7.0), and after concentration, the anti-CLDN-18.2 antibody-drug conjugate ADC-4-MMAE of the present invention was obtained, and the product was stored at -60 to -90°C; wherein m = 2-5, and n = 2-5.

Using the same method as above, the DAR of ADC-4-MMAE measured = 4.55; SEC-HPLC purity was 99.3%.

### Preparation of ADC-2-MMAE (positive control, IMAB362 antibody-drug conjugate, JS107-2-MMAE)

The conjugate of the positive control antibody IMAB362 was prepared using a method similar to the above method. Using the same method as above, the DAR measured = 3.60 and SEC-HPLC was 99.4%.

### Preparation of ADC-5-MMAE (JS107-5-MMAE)

Step 1: The antibody JS012-chi2-hu2-v3-2a (1.0 eq.) was exchanged into the buffer (pH 7.0, 50 mM PB/2 mM EDTA), and TCEP (2.2 eq.) was added to the resulting solution, the temperature was controlled at 25°C, the mixture was reacted for 2 h under stirring to afford a solution containing reduced antibody. Step 2: 10% DMA and vc-MMAE (5.5 eq.) were added to the solution containing reduced antibody, the temperature was controlled at 25°C, and the mixture was reacted for 2 h under stirring. The reaction liquid was exchanged into a buffer solution (50 mM PBS/150 mMNaCl/pH 7.0), and after concentration, the anti-CLDN-18.2 antibody-drug conjugate ADC-5-MMAE of the present invention was obtained, and the product was stored at -60 to -90°C; wherein m = 2-5, and n = 2-5.

Using the same method as above, the DAR of ADC-5-MMAE measured = 4.1; SEC-HPLC purity was 99.5%.

### Preparation of ADC-11-MMAE (JS107-11-MMAE)

Step 1: The antibody JS012-chi2-hu27 (1.0 eq.) was exchanged into the buffer (pH 7.0, 50 mM PB/2 mM EDTA), and TCEP (2.75 eq.) was added to the resulting solution, the temperature was controlled at 25°C, the mixture was reacted for 2 h under stirring to afford a solution containing reduced antibody. Step 2: 10% DMA and vc-MMAE (10.0 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-11-MMAE of the present invention was obtained, and the product was stored at -60 to -90°C; wherein m = 2-5, and n = 2-5.

Using the same method as above, the DAR of ADC-11-MMAE measured = 2.49; SEC-HPLC purity was 92.5%.

### Preparation of ADC-12-MMAE (JS107-12-MMAE)

Step 1: The antibody JS012-chi2-hu29 (1.0 eq.) was exchanged into the buffer (pH 7.0, 50 mM PB/2 mM EDTA), and TCEP (2.5 eq.) was added to the resulting solution, the temperature was controlled at 25°C, the mixture was reacted for 2 h under stirring to afford a solution containing reduced antibody. Step 2: 10% DMA and vc-MMAE (6.0 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-12-MMAE of the present invention was obtained, and the product was stored at -60 to -90°C; wherein m = 2-5, and n = 2-5.

Using the same method as above, the DAR of ADC-12-MMAE measured = 2.22; SEC-HPLC purity was 92.6%.

### Preparation of ADC-13-MMAE (JS107-13-MMAE)

Step 1: The antibody JS012-chi2-hu10 V2 (1.0 eq.) was exchanged into the buffer (pH 7.0, 50 mM PB/2 mM EDTA), and TCEP (2.5 eq.) was added to the resulting solution, the temperature was controlled at 25°C, the mixture was reacted for 2 h under stirring to afford a solution containing reduced antibody. Step 2: 10% DMA and vc-MMAE (6.0 eq.) were added to the solution containing reduced antibody, the temperature was controlled at 25°C, and the mixture was reacted for 2 h under stirring. The reaction liquid was exchanged into a buffer solution (50 mM PBS/150 mMNaCl/pH 7.0), and after concentration, the anti-CLDN-18.2 antibody-drug conjugate ADC-13-MMAE of the present invention was obtained, and the product was stored at -60 to -90°C; wherein m = 2-5, and n = 2-5.

Using the same method as above, the DAR of ADC-13-MMAE measured = 2.37; SEC-HPLC purity was 95.0%.

### Preparation of ADC-14-MMAE (JS107-14-MMAE)

Step 1: The antibody JS012-chi9-hu2 (1.0 eq.) was exchanged into the buffer (pH 7.0, 50 mM PB/2 mM EDTA), and TCEP (2.8 eq.) was added to the resulting solution, the temperature was controlled at 25°C, the mixture was reacted for 2 h under stirring to afford a solution containing reduced antibody. Step 2: 10% DMA and vc-MMAE (6.0 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-14-MMAE of the present invention was obtained, and the product was stored at -60 to -90°C; wherein m = 2-5, and n = 2-5.

Using the same method as above, the DAR of ADC-14-MMAE measured = 2.34; SEC-HPLC purity was 97.0%.

### Preparation of ADC-15-MMAE (JS107-15-MMAE)

Step 1: The antibody JS012-chi9-hu7 V2 (1.0 eq.) was exchanged into the buffer (pH 7.0, 50 mM PB/2 mM EDTA), and TCEP (2.75 eq.) was added to the resulting solution, the temperature was controlled at 25°C, the mixture was reacted for 2 h under stirring to afford a solution containing reduced antibody. Step 2: 10% DMA and vc-MMAE (6.0 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-15-MMAE of the present invention was obtained, and the product was stored at -60 to -90°C; wherein m = 2-5, and n = 2-5.

Using the same method as above, the DAR of ADC-15-MMAE measured = 2.99; SEC-HPLC purity was 94.8%.

### Preparation of ADC-16-MMAE (JS107-16-MMAE)

Step 1: The antibody JS012-chi9-hu8 V2 (1.0 eq.) was exchanged into the buffer (pH 7.0, 50 mM PB/2 mM EDTA), and TCEP (2.2 eq.) was added to the resulting solution, the temperature was controlled at 25°C, the mixture was reacted for 2 h under stirring to afford a solution containing reduced antibody. Step 2: 10% DMA and vc-MMAE (10 eq.) were added to the solution containing reduced antibody, the temperature was controlled at 25°C, and the mixture was reacted for 2 h under stirring. The reaction liquid was exchanged into a buffer solution (50 mM PBS/150 mMNaCl/pH 7.0), and after concentration, the anti-CLDN-18.2 antibody-drug conjugate ADC-16-MMAE of the present invention was obtained, and the product was stored at -60 to -90°C; wherein m = 2-5, and n = 2-5.

Using the same method as above, the DAR of ADC-16-MMAE measured = 2.62; SEC-HPLC purity was 95.2%.

### Preparation of ADC-17-MMAE (JS107-17-MMAE)

Step 1: The antibody JS012-chi10-hu9 (1.0 eq.) was exchanged into the buffer (pH 7.0, 50 mM PB/2 mM EDTA), and TCEP (3.2 eq.) was added to the resulting solution, the temperature was controlled at 25°C, the mixture was reacted for 2 h under stirring to afford a solution containing reduced antibody. Step 2: 10% DMA and vc-MMAE (10 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-17-MMAE of the present invention was obtained, and the product was stored at -60 to -90°C; wherein m = 2-5, and n = 2-5.

Using the same method as above, the DAR of ADC-17-MMAE measured = 3.86; SEC-HPLC purity was 96.1%.

### Preparation of ADC-18-MMAE (JS107-18-MMAE)

Step 1: The antibody JS012-chi27-hu36 (1.0 eq.) was exchanged into the buffer (pH 7.0, 50 mM PB/2 mM EDTA), and TCEP (5.5 eq.) was added to the resulting solution, the temperature was controlled at 25°C, the mixture was reacted for 2 h under stirring to afford a solution containing reduced antibody. Step 2: 10% DMA and vc-MMAE (13 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-18-MMAE of the present invention was obtained, and the product was stored at -60 to -90°C; wherein m = 2-5, and n = 2-5.

Using the same method as above, the DAR of ADC-18-MMAE measured = 2.92; SEC-HPLC purity was 97.1%.

### Preparation of ADC-19-MMAE (JS107-19-MMAE)

Step 1: The antibody JS012-chi27-hu3 V2 (1.0 eq.) was exchanged into the buffer (pH 7.0, 50 mM PB/2 mM EDTA), and TCEP (2.9 eq.) was added to the resulting solution, the temperature was controlled at 25°C, the mixture was reacted for 2 h under stirring to afford a solution containing reduced antibody. Step 2: 10% DMA and vc-MMAE (10 eq.) were added to the solution containing reduced antibody, the temperature was controlled at 25°C, and the mixture was reacted for 2 h under stirring. The reaction liquid was exchanged into a buffer solution (50 mM PBS/150 mMNaCl/pH 7.0), and after concentration, the anti-CLDN-18.2 antibody-drug conjugate ADC-19-MMAE of the present invention was obtained, and the product was stored at -60 to -90°C; wherein m = 2-5, and n = 2-5.

Using the same method as above, the DAR of ADC-19-MMAE measured = 2.85; SEC-HPLC purity was 97.3%.

### 2.2 Anti-CLDN-18.2 antibody was coupled to the linker-cytotoxin MC-MMAF to prepare the conjugate

Drug source: MC-MMAF (linker-cytotoxin, CAS number: 863971-19-1), was purchased from Shanghai Haoyuan Chemexpress Co., Ltd.

The method for preparing anti-CLDN-18.2 antibody conjugated toxin MC-MMAF was as follows:
Step 1: 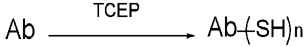
Step 2:

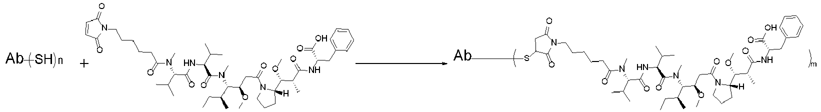

### Preparation of ADC-3-MMAF (JS107-3-MMAF)

Step 1: The antibody JS012-chi9-hu07 (1.0 eq.) was exchanged into the buffer (pH 7.0, 50 mM PB/2 mM EDTA), and TCEP (2.9 eq.) was added to the resulting solution, the temperature was controlled at 25°C, the mixture was reacted for 2 h under stirring to afford a solution containing reduced antibody. Step 2: 10% DMA and MC-MMAF (8.0 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the anti-CLDN-18.2 antibody-drug conjugate ADC-3-MMAF of the present invention was obtained, and the product was stored at -60 to -90°C; wherein m = 2-5, and n = 2-5.

Using the same method as above, the DAR of ADC-3-MMAF measured = 4.13; SEC-HPLC purity was 99.1%.

### Preparation of ADC-4-MMAF (JS107-4-MMAF)

Step 1: The JS012-chi2-hu2-v3-2 (1.0 eq.) solution was exchanged into the buffer (pH 7.0, 50 mM PB/2 mM EDTA), and TCEP (2.9 eq.) was added to the resulting solution, the temperature was controlled at 25°C, the mixture was reacted for 2 h under stirring to afford a solution containing reduced antibody. Step 2: 10% DMA and MC-MMAF (8.0 eq.) were added to the solution containing reduced antibody, the temperature was controlled at 25°C, and the mixture was reacted for 2 h under stirring. The reaction liquid was exchanged into a buffer solution (50 mM PBS/150 mM NaCl/pH 7.0), and after concentration, the ADC-4-MMAF of the present invention was obtained, and the product was stored at -60 to -90°C; wherein m = 2-5, and n = 2-5.

Using the same method as above, the DAR of ADC-4-MMAF measured = 3.85; SEC-HPLC purity was 97.0%.

### Preparation of ADC-2-MMAF (positive control, IMAB362 antibody-drug conjugate)

The conjugate of the positive control antibody IMAB362 was prepared using a method similar to the above method. Using the same method as above, the DAR measured = 3.62 and SEC-HPLC was 98.10%.

### Example 3: Detection of binding activity of anti-CLDN-18.2 antibody-drug conjugate

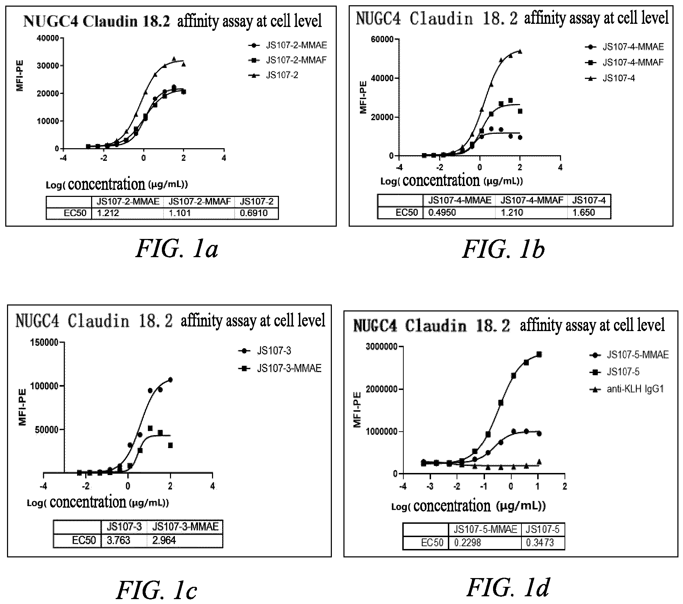

The gastric cancer cell line NUGC4-CLDN-18.2 cells overexpressing human CLDN-18.2 were incubated with the anti-CLDN-18.2 antibody-drug conjugate in a series of gradient dilution concentrations (initial concentration: 100 µg/mL, 3-fold gradient dilution) at 4°C for 30 min, then washed and incubated with a fluorescent labeled secondary antibody. Finally, the fluorescence intensity was detected by BD CantoII flow cytometer. The stronger the fluorescent signal, the higher the affinity of the antibody to the target. The antibody dose-dependent binding curve was fitted by GraphPad (Figs. 1a-1d) and EC50 was calculated. The positive controls were the corresponding monoclonal antibodies, and the negative control was anti-KLH IgG1.

As shown in Figs. 1a-1d, ADC-2-MMAE, ADC-2-MMAF, ADC-3-MMAE, ADC-4-MMAE, ADC-4-MMAF and ADC-5-MMAE could all bind to human CLDN-18.2 which was highly expressed on the surface of gastric cancer cell line NUGC4-CLDN-18.2 cells. Among them, the EC50 value of the binding ability of the drug-conjugates ADC-3-MMAE, ADC-4-MMAE, ADC-4-MMAF and ADC-5-MMAE of the present invention was better than that of the corresponding monoclonal antibodies JS107-3 (JS012-Chi9-hu7), JS107-4 (JS012-Chi2-hu2-v3-2) and JS107-5 (JS012-Chi2-hu2-v3-2a).

### Example 4: Detection of endocytic activity of anti-CLDN-18.2 antibody-drug conjugate

The anti-CLDN-18.2 antibody-drug conjugate was labeled with CypHer 5E Mono NHS Ester dye. When the CypHer5E labeled antibody-drug conjugate binded to Claudin 18.2 on the cell surface, the complex of the conjugate and the antigen would enter the cell nucleus, the CypHer5E dye on the conjugate would release a strong fluorescent signal under the acidic environment in the nucleus, and the release of the fluorescent signal could be detected by FACS detection. The stronger the fluorescence signal, the stronger the endocytic activity of the drug.

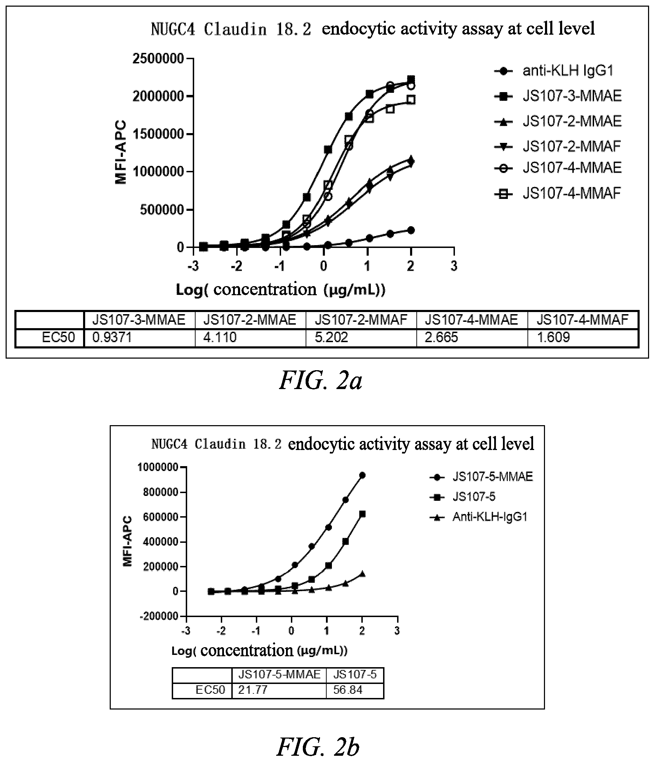

The CypHer5E-labeled anti-CLDN-18.2 antibody-drug conjugate was diluted into a series of gradient dilution concentrations (initial concentration was 100 µg/mL, 3-fold gradient dilution), and incubated with the gastric cancer cell line NUGC4-CLDN-18.2 cells overexpressing human CLDN-18.2 at 37°C for 4 h respectively, washing was performed and the fluorescence intensity was detected by BD CantoII flow cytometry. The antibody dose-dependent binding curve was fitted by GraphPad (Figs. 2a-b) and EC50 was calculated. The control groups were the corresponding monoclonal antibody and anti-KLH IgG1.

As shown in Fig. 2a, ADC-2-MMAE, ADC-2-MMAF, ADC-3-MMAE, ADC-4-MMAE and ADC-4-MMAF can all mediate strong endocytic activity. ADC-3-MMAE, ADC-4-MMAE and ADC-4-MMAF had comparable endocytic activities. ADC-2-MMAE and ADC-2-MMAF had comparable endocytic activities. The endocytic activity of ADC-3-MMAE, ADC-4-MMAE and ADC-4-MMAF was significantly stronger than that of ADC-2-MMAE and ADC-2-MMAF. The results in Fig. 2b showed that the endocytic activity of ADC-5-MMAE was stronger than that of its monoclonal antibody JS107-5 (JS012-Chi2-hu2-v3-2a).

### Example 5: Inhibitory effect of anti-CLDN-18.2 antibody-drug conjugate on cell proliferation

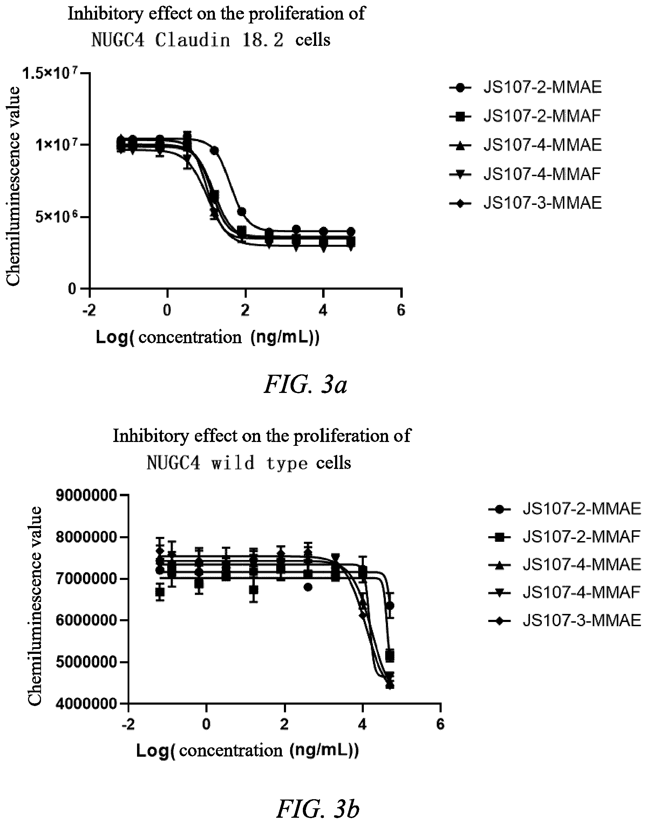

The gastric cancer cell line NUGC4-CLDN-18.2 cells overexpressing human CLDN-18.2 were co-cultured with the anti-CLDN-18.2 antibody-drug conjugate in a series of gradient dilution concentrations (initial concentration: 50 µg/mL, 5-fold gradient dilution) in a cell incubator at 37°C for 48 h respectively, then Cell titer Glo reagent was added, and incubation was performed for 5-10 min at room temperature. Finally, a microplate reader was used to detect the chemiluminescence value (Luminescence). The smaller the chemiluminescence value, the smaller the number of viable cells and the stronger the inhibitory effect of the antibody on cell proliferation. The antibody dose-dependent binding curve was fitted by GraphPad (Figs. 3a-3c) and EC50 was calculated (Table 3). The control group was the corresponding monoclonal antibody.

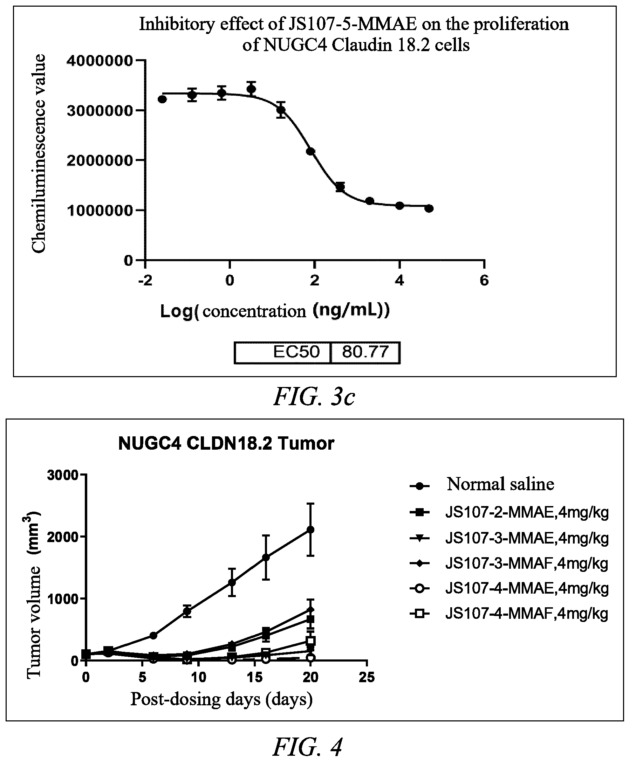

As shown in Figs. 3a-3c, ADC-2-MMAE, ADC-2-MMAF, ADC-3-MMAE, ADC-4-MMAE and ADC-4-MMAF all had the activity of inhibiting the proliferation of the gastric cancer cell line NUGC4-CLDN-18.2 cells overexpressing human CLDN-18.2 (Fig. 3a), and they could inhibit the proliferation of wild-type gastric cancer cell line NUGC4 cells not overexpressing human CLDN-18.2 only at high concentration points (Fig. 3b). As shown in Fig. 3c, ADC-5-MMAE also had the activity of inhibiting the proliferation of gastric cancer cell line NUGC4-CLDN-18.2 cells overexpressing human CLDN-18.2.

As can be seen from Table 3, the inhibitory activity of ADC-2-MMAE on proliferation was weaker than that of ADC-2-MMAF, while the inhibitory activity of ADC-3-MMAE on proliferation was comparable to that of ADC-4-MMAE and ADC-4-MMAF, both of which were better than that of ADC-2-MMAE and ADC-2-MMAF.

**Table 3. Inhibitory effect of anti-CLDN-18.2 antibody-drug conjugate on NUGC4-CLDN-18.2 cells proliferation (IC50)**

| Anti-CLDN-18.2 antibody-drug conjugate | IC50 (ng/mL) |
|---|---|
| ADC-2-MMAE | 45.14 |
| ADC-2-MMAF | 14.79 |
| ADC-4-MMAE | 10.99 |
| ADC-4-MMAF | 9.355 |
| ADC-3-MMAE | 12.70 |

### Example 6: Pharmacological study-1 on the anti-tumor effect of anti-CLDN-18.2 antibody-drug conjugate in the human gastric cancer NUGC4 CLDN-18.2 subcutaneous transplanted tumor model

### 1. Testing purpose

To evaluate the anti-tumor effect of the antibody-drug conjugate of the present invention in the human gastric cancer NUGC4 CLDN-18.2 subcutaneous transplanted tumor model.

### 2. Testing process

130 female BALB/c nude mice ( Shanghai Lingchang Biotechnology Co., Ltd.) aged 6-8 weeks were taken and inoculated subcutaneously on the right back with 2.0 × 10⁶(each/0.1mL) NUGC4 CLDN-18.2 cells. When the average tumor volume was 104 mm³, 36 animals were randomly selected according to the tumor volume and divided into 6 groups, with 6 animals in each group, and the groups were as follows respectively:
normal saline control group (negative control group);
ADC-2-MMAE positive control group, 4 mg/kg;
ADC-3-MMAE treatment group, 4 mg/kg;
ADC-3-MMAF treatment group, 4 mg/kg;
ADC-4-MMAE treatment group, 4 mg/kg;
ADC-4-MMAF treatment group, 4 mg/kg.

Animals in all groups were administrated a single dose intravenously, and the experiment was ended after 20 days of administration. The tumor volume and body weight were measured twice a week, and the body weight and tumor volume of the mice were recorded. At the end of the experiment, the mice were euthanized, and the tumor inhibition rate TGI% (TGI = (1-(Ti-T0)/(Vi-V0)))) was calculated. Ti: the mean tumor volume of the treatment group and the positive control group on day i post-administration; T0: the mean tumor volume of the treatment group and the positive control group on day 0 post-administration; Vi: the mean tumor volume of the negative control group on day i post-administration; V0: the mean tumor volume of the negative control group on day 0 post-administration.

As shown in Fig. 4, at the end of the experiment, the average tumor volume in the normal saline control group was 2113 mm³.

The average tumor volumes of the ADC-2-MMAE positive control group, ADC-3-MMAE treatment group, and ADC-4-MMAE treatment group were 668 mm³, 154 mm³, and 46 mm³, respectively, and the tumor inhibition rates were 71.9%, 97.5% and 102.9% respectively compared with the normal saline control group. It showed that ADC-3-MMAE and ADC-4-MMAE can significantly inhibit the growth of subcutaneous transplanted tumors of human gastric cancer NUGC4 CLDN-18.2 cells at a dose level of 4 mg/kg, and the inhibitory effect was better than the positive control ADC-2-MMAE.

The average tumor volumes of the ADC-3-MMAF treatment group and the ADC-4-MMAF treatment group were 824 mm³ and 319 mm³ respectively, and the tumor inhibition rates were 64.2% and 89.3% respectively compared with the normal saline control group. It showed that ADC-3-MMAF and ADC-4-MMAF can significantly inhibit the growth of subcutaneous transplanted tumors of human gastric cancer NUGC4 CLDN-18.2 cells at a dose level of 4 mg/kg.

### Example 7: Pharmacological study-2 on the anti-tumor effect of anti-CLDN-18.2 antibody-drug conjugate in the human gastric cancer NUGC4 CLDN-18.2 subcutaneous transplanted tumor model

### 1. Testing purpose

To evaluate the anti-tumor effect of the antibody-drug conjugate of the present invention in the human gastric cancer NUGC4 CLDN-18.2 subcutaneous transplantation model.

### 2. Testing process

130 female BALB/c nude mice ( Shanghai Lingchang Biotechnology Co., Ltd.) aged 6-7 weeks were taken and inoculated subcutaneously on the right back with 2.0 × 10⁶(each/0.1mL) NUGC4 CLDN-18.2 cells. When the average tumor volume was 104 mm³, 42 animals were randomly selected according to the tumor volume and divided into 7 groups, with 6 animals in each group, and the groups were as follows respectively:
normal saline control group (negative control group);
ADC-2-MMAE positive control group, 2 mg/kg;
ADC-2-MMAE positive control group, 3 mg/kg;
ADC-4-MMAE treatment group, 2 mg/kg;
ADC-4-MMAE treatment group, 3 mg/kg;
ADC-5-MMAE treatment group, 2 mg/kg;
ADC-5-MMAE treatment group, 3 mg/kg.

Animals in all groups were administrated a single dose intravenously, and the experiment was ended after 21 days of administration. The tumor volume and body weight were measured twice a week, and the body weight and tumor volume of the mice were recorded. At the end of the experiment, the mice were euthanized, and the tumor inhibition rate TGI% (TGI = (1-(Ti-T0)/(Vi-V0))) was calculated. Ti: the mean tumor volume of the treatment group and the positive control group on day i post-administration; T0: the mean tumor volume of the treatment group and the positive control group on day 0 post-administration; Vi: the mean tumor volume of the negative control group on day i post-administration; V0: the mean tumor volume of the negative control group on day 0 post-administration.

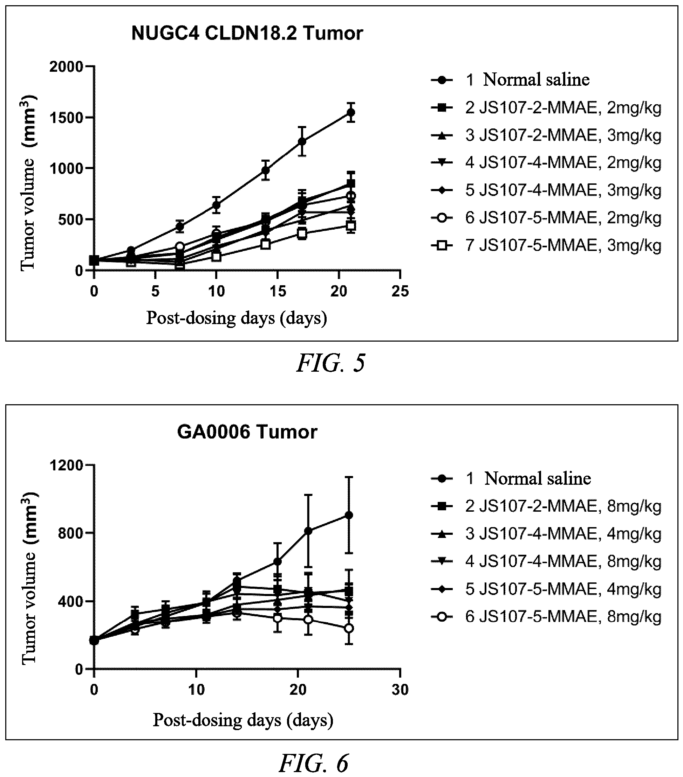

As shown in Fig. 5, at the end of the experiment, the average tumor volume in the normal saline control group was 1549 mm³.

The results for the positive control group and the treatment group were shown in Fig. 5 and summarized below.

| Test group | Average tumor volume | Tumor inhibition rate |
|---|---|---|
| ADC-2-MMAE, 2 mg/kg positive control group | 851 mm³ | 48.1% |
| ADC-2-MMAE, 3 mg/kg positive control group | 633 mm³ | 63.1% |
| ADC-4-MMAE, 2 mg/kg treatment group | 834 mm³ | 49.3% |

| | | |
|---|---|---|
| ADC-4-MMAE, 3 mg/kg treatment group | 568 mm³ | 67.6% |
| ADC-5-MMAE, 2 mg/kg treatment group | 729 mm³ | 56.5% |
| ADC-5-MMAE, 3 mg/kg treatment group | 438 mm³ | 76.6% |

It showed that ADC-4-MMAE and ADC-5-MMAE could significantly inhibit the growth of subcutaneous transplanted tumors of human gastric cancer NUGC4 CLDN-18.2 cells at dose levels of 2 and 3 mg/kg, and had good dose effects which both were better than that of positive control ADC-2-MMAE respectively.

### Example 8: Pharmacological study on the anti-tumor effect of anti-CLDN-18.2 antibody-drug conjugate in the human gastric cancer GA0006 subcutaneous transplanted tumor model

### 1. Testing purpose

To evaluate the anti-tumor effect of the antibody-drug conjugate of the present invention in the human gastric cancer GA0006 subcutaneous transplantation model.

### 2. Testing process

87 female BALB/c nude mice aged 4-8 week (Jiangsu Jicui Gempharmatech Co., Ltd.) were taken and inoculated subcutaneously on the right back with GA0006 tumor mass with a diameter of 3 × 3 × 3 mm³. When the average tumor volume was 169.57 mm³, 36 animals were randomly selected and divided into 6 groups according to tumor size, with 6 animals in each group, and the groups were as follows respectively:
normal saline control group (negative control group);
ADC-2-MMAE positive control group, 8 mg/kg;
ADC-4-MMAE treatment group, 4 mg/kg;
ADC-4-MMAE treatment group, 8 mg/kg;
ADC-5-MMAE treatment group, 4 mg/kg;
ADC-5-MMAE treatment group, 8 mg/kg.

Animals in all groups were administrated a single dose intravenously, and the experiment was ended after 25 days of administration. The tumor volume and body weight were measured twice a week, and the body weight and tumor volume of the mice were recorded. At the end of the experiment, the mice were euthanized, and the tumor inhibition rate TGI% (TGI = 1-T/C (%)) was calculated. T/C (%) was the tumor proliferation rate, that is, the ratio of tumor volume between the treatment group and the control group in percentage at a certain time point. T and C were the tumor volumes (TV) of the treatment group and the control group at a specific time point, respectively.

As shown in Fig. 6, at the end of the experiment, the average tumor volume in the normal saline control group was 904.54 mm³. The results for the positive control group and the treatment group were shown in Fig. 6 and summarized below.

| Test group | Average tumor volume | Tumor inhibition rate |
|---|---|---|
| ADC-2-MMAE, 8 mg/kg positive control group | 461.11 mm³ | 49.52% |
| ADC-4-MMAE, 4 mg/kg treatment group | 470.58 mm³ | 41.96% |
| ADC-4-MMAE, 8 mg/kg treatment group | 400.18 mm³ | 55.74% |
| ADC-5-MMAE, 4 mg/kg treatment group | 363.22 mm³ | 57.03% |
| ADC-5-MMAE, 8 mg/kg treatment group | 239.88 mm³ | 74.10% |

The results showed that both of ADC-4-MMAE and ADC-5-MMAE could significantly inhibit the growth of subcutaneous transplanted tumors of human gastric cancer GA0006 cells at the dose levels of 4 mg/kg and 8 mg/kg, and the tumor inhibitory effect was comparable to or better than that of the positive control ADC-2-MMAE.

## Claims

1. An anti-CLDN-18.2 antibody-drug conjugate (ADC) with a structure as shown in formula (I-1), or a pharmaceutically acceptable salt thereof:
Ab-(L-D)m formula (I-1)
**characterized in that**,
L is a linker unit;
D is a small molecule drug with cytotoxicity;
m represents the average number of L-D units conjugated to Ab, and m is selected from 1-8, preferably 2-7, and more preferably 2-5;
Ab is an anti-CLDN-18.2 antibody or an antigen-binding fragment thereof, which comprises
(I) a heavy chain variable region comprising HCDR1 and HCDR2 with amino acid sequences as shown in SEQ ID NO: 1 and SEQ ID NO: 72, respectively, and HCDR3 as shown in SEQ ID NO: 73 or 3; and a light chain variable region comprising LCDR1, LCDR2 and LCDR3 with amino acid sequences as shown in SEQ ID NO: 7, SEQ ID NO: 8 and SEQ ID NO: 9, respectively; or
(II) a heavy chain variable region comprising HCDR1, HCDR2 and HCDR3 with amino acid sequences as shown in SEQ ID NO: 1, SEQ ID NO: 41 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(III) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(IV) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 19, SEQ ID NO: 20 and SEQ ID NO: 21, respectively; or
(V) a heavy chain variable region comprising HCDR1 and HCDR2 with amino acid sequences as shown in SEQ ID NO: 28 and SEQ ID NO: 74, respectively, and HCDR3 as shown in SEQ ID NO: 30 or 75; and a light chain variable region comprising LCDR1, LCDR2 and LCDR3 with amino acid sequences as shown in SEQ ID NO: 13, SEQ ID NO: 14 and SEQ ID NO: 15, respectively.

2. The anti-CLDN-18.2 antibody-drug conjugate or the pharmaceutically acceptable salt thereof according to claim 1, **characterized in that** the small molecule drug D is monomethyl auristatin or an alkaloid maytansine; preferably, the monomethyl auristatin is monomethyl auristatin E (MMAE) or monomethyl auristatin F (MMAF), and the maytansine is N2'-deacetyl-N2'-(3-mercapto-1-oxopropyl)-maytansine (DM1), N2'-deacetyl-N2'-(4-mercapto-1-oxopentyl)-maytansine (DM3) or N2'-deacetyl-N2'-(4-mercapto-4-methyl-1-oxopentyl)-maytansine (DM4).

3. The anti-CLDN-18.2 antibody-drug conjugate or the pharmaceutically acceptable salt thereof according to claim 1, **characterized in that** the L is selected from N-succinimidyl 4-(2-pyridyldithio)pentanoate (SPP), N-succinimidyl (4-iodoacetyl)aminobenzoate (SIAB), N-succinimidyl 4-(maleimidomethyl)cyclohexanecarboxylate (SMCC), 6-maleimidocaproyl (MC), maleimidopropionyl (MP), valine-citrulline (VC), alanine-phenylalanine (ala-phe), p-aminobenzyloxycarbonyl (PAB) and MC-VC-PAB; preferably, the L is 6-maleimidocaproyl (MC) or MC-VC-PAB.

4. The anti-CLDN-18.2 antibody-drug conjugate or the pharmaceutically acceptable salt thereof according to any one of claims 1-3, **characterized in that** the anti-CLDN-18.2 antibody-drug conjugate is as represented by the following formula: 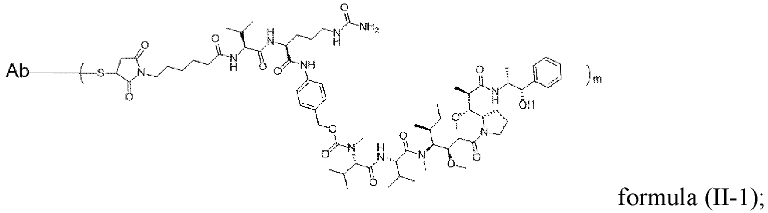 or 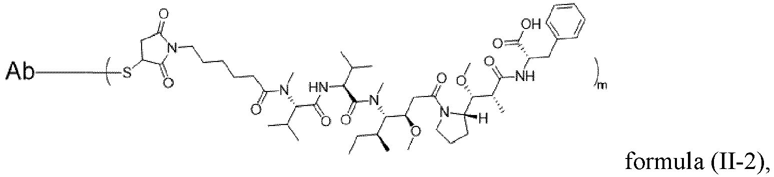 wherein m and Ab are as defined in claim 1.

5. The anti-CLDN-18.2 antibody-drug conjugate or the pharmaceutically acceptable salt thereof according to any one of claims 1-4, **characterized in that** the anti-CLDN-18.2 antibody or antigen-binding fragment thereof comprises:
(I) a heavy chain variable region with an amino acid sequence as shown in or having at least 95%, 96%, 97%, 98% or 99% identity to any one of SEQ ID NO: 55, 57, 82 or 83, and a light chain variable region with an amino acid sequence as shown in or having at least 95%, 96%, 97%, 98% or 99% identity to SEQ ID NO: 56; or
(II) a heavy chain variable region with an amino acid sequence as shown in or having at least 95%, 96%, 97%, 98% or 99% identity to any one of SEQ ID NO: 60, 76 or 78, and a light chain variable region with an amino acid sequence as shown in or having at least 95%, 96%, 97%, 98% or 99% identity to SEQ ID NO: 61; or
(III) a heavy chain variable region with an amino acid sequence as shown in or having at least 95%, 96%, 97%, 98% or 99% identity to SEQ ID NO: 76, and a light chain variable region with an amino acid sequence as shown in or having at least 95%, 96%, 97%, 98% or 99% identity to SEQ ID NO: 77; or
(IV) a heavy chain variable region with an amino acid sequence as shown in or having at least 95%, 96%, 97%, 98% or 99% identity to SEQ ID NO: 79; and a light chain variable region with an amino acid sequence as shown in or having at least 95%, 96%, 97%, 98% or 99% identity to SEQ ID NO: 80; or
(V) a heavy chain variable region with an amino acid sequence as shown in or having at least 95%, 96%, 97%, 98% or 99% identity to SEQ ID NO: 55, and a light chain variable region with an amino acid sequence as shown in or having at least 95%, 96%, 97%, 98% or 99% identity to SEQ ID NO: 81.

6. The anti-CLDN-18.2 antibody-drug conjugate or the pharmaceutically acceptable salt thereof according to any one of claims 1-5, **characterized in that** the anti-CLDN-18.2 antibody comprises:
(1) a heavy chain with an amino acid sequence as shown in or having at least 95%, 96%, 97%, 98% or 99% identity to SEQ ID NO: 64, and a light chain with an amino acid sequence as shown in or having at least 95%, 96%, 97%, 98% or 99% identity to SEQ ID NO: 65; or
(2) a heavy chain with an amino acid sequence as shown in or having at least 95%, 96%, 97%, 98% or 99% identity to SEQ ID NO: 68; and a light chain with an amino acid sequence as shown in or having at least 95%, 96%, 97%, 98% or 99% identity to SEQ ID NO: 69; or
(3) a heavy chain with an amino acid sequence as shown in or having at least 95%, 96%, 97%, 98% or 99% identity to SEQ ID NO: 84; and a light chain with an amino acid sequence as shown in or having at least 95%, 96%, 97%, 98% or 99% identity to SEQ ID NO: 85; or
(4) a heavy chain with an amino acid sequence as shown in or having at least 95%, 96%, 97%, 98% or 99% identity to SEQ ID NO: 86; and a light chain with an amino acid sequence as shown in or having at least 95%, 96%, 97%, 98% or 99% identity to SEQ ID NO: 87; or
(5) a heavy chain with an amino acid sequence as shown in or having at least 95%, 96%, 97%, 98% or 99% identity to SEQ ID NO: 88; and a light chain with an amino acid sequence as shown in or having at least 95%, 96%, 97%, 98% or 99% identity to SEQ ID NO: 89; or
(6) a heavy chain with an amino acid sequence as shown in or having at least 95%, 96%, 97%, 98% or 99% identity to SEQ ID NO: 90; and a light chain with an amino acid sequence as shown in or having at least 95%, 96%, 97%, 98% or 99% identity to SEQ ID NO: 91; or
(7) a heavy chain with an amino acid sequence as shown in or having at least 95%, 96%, 97%, 98% or 99% identity to SEQ ID NO: 92; and a light chain with an amino acid sequence as shown in or having at least 95%, 96%, 97%, 98% or 99% identity to SEQ ID NO: 93; or
(8) a heavy chain with an amino acid sequence as shown in or having at least 95%, 96%, 97%, 98% or 99% identity to SEQ ID NO: 94; and a light chain with an amino acid sequence as shown in or having at least 95%, 96%, 97%, 98% or 99% identity to SEQ ID NO: 95; or
(9) a heavy chain with an amino acid sequence as shown in or having at least 95%, 96%, 97%, 98% or 99% identity to SEQ ID NO: 96; and a light chain with an amino acid sequence as shown in or having at least 95%, 96%, 97%, 98% or 99% identity to SEQ ID NO: 97; or
(10) a heavy chain with an amino acid sequence as shown in or having at least 95%, 96%, 97%, 98% or 99% identity to SEQ ID NO: 98; and a light chain with an amino acid sequence as shown in or having at least 95%, 96%, 97%, 98% or 99% identity to SEQ ID NO: 99; or
(11) a heavy chain with an amino acid sequence as shown in or having at least 95%, 96%, 97%, 98% or 99% identity to SEQ ID NO: 100; and a light chain with an amino acid sequence as shown in or having at least 95%, 96%, 97%, 98% or 99% identity to SEQ ID NO: 101.

7. The anti-CLDN-18.2 antibody-drug conjugate or the pharmaceutically acceptable salt thereof according to any one of claims 1-6, **characterized in that** the antibody or antigen-binding fragment is a murine antibody, a chimeric antibody, a humanized antibody, or a fully human antibody.

8. The anti-CLDN-18.2 antibody-drug conjugate or the pharmaceutically acceptable salt thereof according to any one of claims 1-7, **characterized in that** the antibody or antigen-binding fragment is Fab, Fab', Fab'-SH, Fv, scFv, F(ab')2, or sdAb.

9. The anti-CLDN-18.2 antibody-drug conjugate or the pharmaceutically acceptable salt thereof according to any one of claims 1-9, **characterized in that** the antibody is of any IgG subtype, such as IgG1, IgG2, IgG3 or IgG4; preferably, the antibody is hypofucosylated or afucosylated.

10. A pharmaceutical composition, **characterized in that** the pharmaceutical composition comprises the anti-CLDN-18.2 antibody-drug conjugate or the pharmaceutically acceptable salt thereof according to any one of claims 1-9, and a pharmaceutically acceptable carrier or excipient.

11. The antibody-drug conjugate or the pharmaceutically acceptable salt thereof according to any one of claims 1-9 or the pharmaceutical composition according to claim 10 for use in the preparation of a medicament for the treatment and/or prevention of a disease or condition mediated by CLDN-18.2, wherein preferably, the disease or condition is a cancer; and more preferably, the cancer is selected from gastric cancer, esophageal cancer, gastroesophageal cancer, pancreatic cancer, cholangiocarcinoma, lung cancer, ovarian cancer, colon cancer, liver cancer, head and neck cancer, gallbladder cancer, intestinal cancer, and bladder cancer.

12. A method of treating and/or preventing a disease or condition mediated by CLDN-18.2, **characterized in that** the method comprises administering the antibody-drug conjugate according to any one of claims 1-9 or the pharmaceutical composition according to claim 10 to a subject in need thereof, wherein preferably, the disease or condition is a cancer; and more preferably, the cancer is selected from gastric cancer, esophageal cancer, gastroesophageal cancer, pancreatic cancer, cholangiocarcinoma, lung cancer, ovarian cancer, colon cancer, liver cancer, head and neck cancer, gallbladder cancer, intestinal cancer, and bladder cancer.

13. A pharmaceutical combination, **characterized in that** the pharmaceutical combination comprises the antibody-drug conjugate or the pharmaceutically acceptable salt thereof according to any one of claims 1-9 or the pharmaceutical composition according to claim 10, and one or more additional therapeutic agents.

14. A kit, **characterized in that** the kit comprises the antibody-drug conjugate according to any one of claims 1-9, or the pharmaceutical composition according to claim 10.
